# EUROPEAN PATENT SPECIFICATION

(11) **EP 2 783 007 B1**
(45) Date of publication and mention of the grant of the patent: **16.08.2017**
(21) Application number: 12790547.9
(22) Date of filing: 21.11.2012
(51) Int. Cl.: C12P 7/16

(54) **MICROORGANISM STRAINS FOR THE PRODUCTION OF 2,3-BUTANEDIOL**
MIKROORGANISMUSSTÄMME ZUR HERSTELLUNG VON 2,3-BUTANDIOL
SOUCHES DE MICRO-ORGANISMES POUR LA PRODUCTION DE 2,3-BUTANEDIOL

(30) Priority: 21.11.2011 EP 11190018; 21.11.2011 US 201161562136 P
(43) Date of publication of application: 01.10.2014
(73) Proprietor: Metabolic Explorer, 63360 Saint Beauzire (FR)
(72) Inventor: DISCHERT, Wanda, 63270 Vic-Le-Comte (FR); LETELLIER, Guillaume, 63530 Volvic (FR); FIGGE, Rainer, 63450 Le Crest (FR); DOUCHIN, Véronique, 63000 Clermont Ferrand (FR)
(74) Representative: Regimbeau
(86) International application number: PCT/EP2012/073242
(87) International publication number: WO 2013/076144

(56) References cited:
- WO-A1-2011/041426
- WO-A1-2012/104244
- WO-A2-2011/040901
- WO-A2-2012/124890
- EHSANI MARYAM ET AL: "Engineering of 2,3-Butanediol Dehydrogenase To Reduce Acetoin Formation by Glycerol-Overproducing, Low-Alcohol Saccharomyces cerevisiae", APPLIED AND ENVIRONMENTAL MICROBIOLOGY, AMERICAN SOCIETY FOR MICROBIOLOGY, US, vol. 75, no. 10, 1 May 2009 (2009-05-01), pages 3196-3205, XP002564242, ISSN: 0099-2240, DOI: 10.1128/AEM.02157-08

### SEQUENCE LISTING

<110> METABOLIC EXPLORER
<120> MICROORGANISM STRAINS FOR THE PRODUCTION OF 2,3-BUTANEDIOL
<130> 361606
<150> EP11190018.9
   <151> 2011-11-21
<150> US61/562,136
   <151> 2011-11-21
<160> 56
<170> PatentIn version 3.5
<210> 1
   <211> 99
   <212> DNA
   <213> Artificial Sequence
<220>
   <223> Oligonucleotide
<400> 1
   ccttagccag tttgttttcg ccagttcgat cacttcatca ccgcgtccgc tgatgattgc        60
   gcgcagcata tacaggctgc atatgaatat cctccttag                               99
<210> 2
   <211> 102
   <212> DNA
   <213> Artificial Sequence
<220>
   <223> Oligonucleotide
<400> 2
   cggttgcagc ttatatcgcc aaaacactcg aatcggcagg ggtgaaacgc atctggggag        60
   tcacaggcga ctctctgaac ggtgtaggct ggagctgctt cg                          102
<210> 3
   <211> 101
   <212> DNA
   <213> Artificial Sequence
<220>
   <223> Oligonucleotide
<400> 3
   ttaaaccagt tcgttcgggc aggtttcgcc tttttccaga ttgcttaagt tttgcagcgt 60
   agtctgagaa atactggtca gcatatgaat atcctcctta g 101
<210> 4
   <211> 100
   <212> DNA
   <213> Artificial Sequence
<220>
   <223> Oligonucleotide
<400> 4
   gaaactcgcc gtttatagca caaaacagta cgacaagaag tacctgcaac aggtgaacga 60
   gtcctttggc tttgagctgg tgtaggctgg agctgcttcg 100
<210> 5
   <211> 100
   <212> DNA
   <213> Artificial Sequence
<220>
   <223> Oligonucleotide
<400> 5
   cgagtaagtt agtactggtt ctgaactgcg gtagttcttc actgaaattt gccatcatcg 60
   atgcagtaaa tggtgaagag tgtaggctgg agctgcttcg 100
<210> 6
   <211> 97
   <212> DNA
   <213> Artificial Sequence
<220>
   <223> Oligonucleotide
<400> 6
   gctgctgtgc agactgaatc gcagtcagcg cgatggtgta gacgatatcg tcaaccagtg 60
   cgccacggga caggtcgcat atgaatatcc tccttag 97
<210> 7
   <211> 104
   <212> DNA
   <213> Artificial Sequence
<220>
   <223> Oligonucleotide
<400> 7
   ccggagctcg agctgttgac aattaatcat ccggctcgta taatgtgtgg aagtcgacgt 60
   taaccctagg taaggaggtt ataaatgtct gaaattactt tggg 104
<210> 8
   <211> 38
   <212> DNA
   <213> Artificial Sequence
<220>
   <223> Oligonucleotide
<400> 8
   cgcggatccg cggctagctt attgcttagc gttggtag 38
<210> 9
   <211> 107
   <212> DNA
   <213> Artificial Sequence
<220>
   <223> Oligonucleotide
<400> 9
   cgggagctcg agctgttgac aattaatcat ccggctcgta taatgtgtgg aagtcgacgt 60
   taaccctagg taaggaggtt ataaatgaaa aaagtcgcac ttgttac 107
<210> 10
   <211> 28
   <212> DNA
   <213> Artificial Sequence
<220>
   <223> Oligonucleotide
<400> 10
   gctctagatt agttaaacac catcccgc 28
<210> 11
   <211> 86
   <212> DNA
   <213> Artificial Sequence
<220>
   <223> Oligonucleotide
<400> 11
   ggactagtga gctgttgaca attaatcatc cggctcgtat aatgtgtgga ataaggaggt 60
   tataaatgtc cgagcttaat gaaaag 86
<210> 12
   <211> 28
   <212> DNA
   <213> Artificial Sequence
<220>
   <223> Oligonucleotide
<400> 12
   cggaattctt acatagattg agtgaagg 28
<210> 13
   <211> 100
   <212> DNA
   <213> Artificial Sequence
<220>
   <223> Oligonucleotide
<400> 13
   atctatgtaa gaattcgagc tgttgacaat taatcatccg gctcgtataa tgtgtggaat 60
   aaggaggtta taaatgagta agcgtaaagt cgccattatc 100
<210> 14
   <211> 68
   <212> DNA
   <213> Artificial Sequence
<220>
   <223> Oligonucleotide
<400> 14
   aaaagctggg taccgggccc gcagaaaggc ccacccgaag gtgagccagt catgccgctt 60
   ctcctgcc 68
<210> 15
   <211> 103
   <212> DNA
   <213> Artificial Sequence
<220>
   <223> Oligonucleotide
<400> 15
   ctaaacgtag aacctgtctt attgagcttt ccggcgagag ttcaatgggt cacactggct 60
   caccttcggg tgggcctttc tgccatatga atatcctcct tag 103
<210> 16
   <211> 121
   <212> DNA
   <213> Artificial Sequence
<220>
   <223> Oligonucleotide
<400> 16
   cgttctgaca tgggttattc cttatctatc taataacgtt gagttttctg gaacctgttt 60
   ccacacagta tacgagccgg atgattaatc gacaacagct ctgtaggctg gagctgcttc 120
   g 121
<210> 17
   <211> 85
   <212> DNA
   <213> Artificial Sequence
<220>
   <223> Oligonucleotide
<400> 17
   cgggatccga gctgttgaca attaatcatc cggctcgtat aatgtgtgga ataaggaggt 60
   tataaatgga taaacagtat ccggt 85
<210> 18
   <211> 57
   <212> DNA
   <213> Artificial Sequence
<220>
   <223> Oligonucleotide
<400> 18
   cggaattcgc agaaaggccc acccgaaggt gagccagtta cagaatctgt gacagat 57
<210> 19
   <211> 108
   <212> DNA
   <213> Artificial Sequence
<220>
   <223> Oligonucleotide
<400> 19
   cgggagctcg agctgttgac aattaatcat ccggctcgta taatgtgtgg aagtcgacgt 60
   taaccctagg taaggaggtt ataaatgctg cgtagcctgc tgcaaagc 108
<210> 20
   <211> 58
   <212> DNA
   <213> Artificial Sequence
<220>
   <223> Oligonucleotide
<400> 20
   gcggtaccgc agaaaggccc acccgaaggt gagccagtta acccggaggc agctggct 58
<210> 21
   <211> 111
   <212> DNA
   <213> Artificial Sequence
<220>
   <223> Oligonucleotide
<400> 21
   gaatgcgacc aataactgac atttataacc tccttaggat ccgtcgactt ccacacatta 60
   tacgagccgg atgattaatt gtcaacagct ccatatgaat atcctcctta g 111
<210> 22
   <211> 120
   <212> DNA
   <213> Artificial Sequence
<220>
   <223> Oligonucleotide
<400> 22
   ctatctatac tttaaggtga ctgccaaaac agactcgacg tagccttcga gctgcgcacc 60
   aacacggcct cagatgggcc acatctggag aaacaccgca tgtaggctgg agctgcttcg 120
<210> 23
   <211> 20
   <212> DNA
   <213> Artificial Sequence
<220>
   <223> Oligonucleotide
<400> 23
   gtcagttatt ggtcgcattc 20
<210> 24
   <211> 19
   <212> DNA
   <213> Artificial Sequence
<220>
   <223> Oligonucleotide
<400> 24
   ctcgtcgttc atctgtttg 19
<210> 25
   <211> 26
   <212> DNA
   <213> Artificial Sequence
<220>
   <223> Oligonucleotide
<400> 25
   gctatctata ctttaaggtg actgcc 26
<210> 26
   <211> 115
   <212> DNA
   <213> Artificial Sequence
<220>
   <223> Oligonucleotide
<400> 26
   atctatgtaa gaattcgcta gcgagctgtt gacaattaat catccggctc gtataatgtg 60
   tggaagaacc ctaaatagaa ggaggcgcac aaaatgaaat tgttaaaacg agaag 115
<210> 27
   <211> 71
   <212> DNA
   <213> Artificial Sequence
<220>
   <223> Oligonucleotide
<400> 27
   cggtatcgat aagcttgcag aaaggcccac ccgaaggtga gccagtacgt attaattcaa 60
   tgccggctcg c 71
<210> 28
   <211> 62
   <212> DNA
   <213> Artificial Sequence
<220>
   <223> Oligonucleotide
<400> 28
   tcaagctata ccaagcatac aatcaactat ctcatataca gcataggcca ctagtggatc 60
   tg 62
<210> 29
   <211> 60
   <212> DNA
   <213> Artificial Sequence
<220>
   <223> Oligonucleotide
<400> 29
   cttatttaat aataaaaatc ataaatcata agaaattcgc ccagctgaag cttcgtacgc 60
<210> 30
   <211> 62
   <212> DNA
   <213> Artificial Sequence
<220>
   <223> Oligonucleotide
<400> 30
   gttaaaacta ggaatagtat agtcataagt taacaccatc gcataggcca ctagtggatc 60
   tg 62
<210> 31
   <211> 60
   <212> DNA
   <213> Artificial Sequence
<220>
   <223> Oligonucleotide
<400> 31
   acaaagactt tcataaaaag tttgggtgcg taacacgcta ccagctgaag cttcgtacgc 60
<210> 32
   <211> 62
   <212> DNA
   <213> Artificial Sequence
<220>
   <223> Oligonucleotide
<400> 32
   aagaaaatta tttaactaca tatctacaaa atcaaagcat gcataggcca ctagtggatc 60
   tg 62
<210> 33
   <211> 60
   <212> DNA
   <213> Artificial Sequence
<220>
   <223> Oligonucleotide
<400> 33
   taaaaagtaa aaatatattc atcaaattcg ttacaaaaga ccagctgaag cttcgtacgc 60
<210> 34
   <211> 70
   <212> DNA
   <213> Artificial Sequence
<220>
   <223> Oligonucleotide
<400> 34
   ctaaaccctt tgagctaaga ggagataaat acaacagaat caattttcaa ttcgtacgct 60
   gcaggtcgac 70
<210> 35
   <211> 70
   <212> DNA
   <213> Artificial Sequence
<220>
   <223> Oligonucleotide
<400> 35
   gtctgcattt tttactgaaa atgcttttga aataaatgtt tttgaaatgc ataggccact 60
   agtggatctg 70
<210> 36
   <211> 65
   <212> DNA
   <213> Artificial Sequence
<220>
   <223> Oligonucleotide
<400> 36
   gaataaacac aaaaacaaaa agttttttta attttaatca aaaaatgaga gctttggcat 60
   atttc 65
<210> 37
   <211> 67
   <212> DNA
   <213> Artificial Sequence
<220>
   <223> Oligonucleotide
<400> 37
   cggatgtggg gggagggcgt gaatgtaagc gtgacataac taattttact tcatttcacc 60
   gtgattg 67
<210> 38
   <211> 73
   <212> DNA
   <213> Artificial Sequence
<220>
   <223> Oligonucleotide
<400> 38
   agaataaaca caaaaacaaa aagttttttt aattttaatc aaaaaatgag taagcgtaaa 60
   gtcgccatta tcg 73
<210> 39
   <211> 65
   <212> DNA
   <213> Artificial Sequence
<220>
   <223> Oligonucleotide
<400> 39
   gagggcgtga atgtaagcgt gacataacta attacatgac tcgagtcatg ccgcttctcc 60
   tgcct 65
<210> 40
   <211> 67
   <212> DNA
   <213> Artificial Sequence
<220>
   <223> Oligonucleotide
<400> 40
   gaataaacac aaaaacaaaa agttttttta attttaatca aaaaaatgtc agttattggt 60
   cgcattc 67
<210> 41
   <211> 65
   <212> DNA
   <213> Artificial Sequence
<220>
   <223> Oligonucleotide
<400> 41
   gagggcgtga atgtaagcgt gacataacta attacatgac tcgagttaga acattacctt 60
   atgac 65
<210> 42
   <211> 63
   <212> DNA
   <213> Artificial Sequence
<220>
   <223> Oligonucleotide
<400> 42
   tcaaggaagt aattatctac tttttacaac aaatataaaa caatgtccga gcttaatgaa 60
   aag 63
<210> 43
   <211> 66
   <212> DNA
   <213> Artificial Sequence
<220>
   <223> Oligonucleotide
<400> 43
   aatgcttata aaactttaac taataattag agattaaatc gcttacatag attgagtgaa 60
   ggtacg 66
<210> 44
   <211> 27
   <212> DNA
   <213> Artificial Sequence
<220>
   <223> Oligonucleotide
<400> 44
   aattagttat gtcacgctta cattcac 27
<210> 45
   <211> 65
   <212> DNA
   <213> Artificial Sequence
<220>
   <223> Oligonucleotide
<400> 45
   ggttttttca gttttgttct ttttgcaaac agcggccgcc taaggggcga attgggtacc 60
   ggccg 65
<210> 46
   <211> 32
   <212> DNA
   <213> Artificial Sequence
<220>
   <223> Oligonucleotide
<400> 46
   taagcgattt aatctctaat tattagttaa ag 32
<210> 47
   <211> 68
   <212> DNA
   <213> Artificial Sequence
<220>
   <223> Oligonucleotide
<400> 47
   ctatgcggtg tgaaataccg cacagatgcg taaggagaaa atacctgttc cttaatcaag 60
   gatacctc 68
<210> 48
   <211> 37
   <212> DNA
   <213> Artificial Sequence
<220>
   <223> Oligonucleotide
<400> 48
   ccttaggcgg ccgctgtttg caaaaagaac aaaactg 37
<210> 49
   <211> 37
   <212> DNA
   <213> Artificial Sequence
<220>
   <223> Oligonucleotide
<400> 49
   cattgtttta tatttgttgt aaaaagtaga taattac 37
<210> 50
   <211> 69
   <212> DNA
   <213> Artificial Sequence
<220>
   <223> Oligonucleotide
<400> 50
   gaataaacac aaaaacaaaa agttttttta attttaatca aaaaaatgaa attgttaaaa 60
   cgagaaggc 69
<210> 51
   <211> 70
   <212> DNA
   <213> Artificial Sequence
<220>
   <223> Oligonucleotide
<400> 51
   cggatgtggg gggagggcgt gaatgtaagc gtgacataac taattttaca ttcctccttt 60
   ttcatatgac 70
<210> 52
   <211> 62
   <212> DNA
   <213> Artificial Sequence
<220>
   <223> Oligonucleotide
<400> 52
   catcaaggaa gtaattatct actttttaca acaaatataa aacaatggcg agagtcataa 60
   gc 62
<210> 53
   <211> 66
   <212> DNA
   <213> Artificial Sequence
<220>
   <223> Oligonucleotide
<400> 53
   cataaaaatg cttataaaac tttaactaat aattagagat taaatcgctt aattcaatgc 60
   cggctc 66
<210> 54
   <211> 771
   <212> DNA
   <213> Klebsiella pneumoniae
<400> 54
   atgaaaaaag tcgcacttgt taccggcgcc ggccagggga ttggtaaagc tatcgccctt 60
   cgtctggtga aggatggatt tgccgtggcc attgccgatt ataacgacac caccgccaaa 120
   gcggtcgcct ccgaaatcaa ccaggccggc ggccgcgcca tggcggtgaa agtggatgtc 180
   tccgaccgcg atcaggtgtt tgccgccgtc gaacaggcgc gcaaaacgct gggcggcttc 240
   gacgtcatcg tcaacaacgc cggcgtggcg ccgtccacgc cgatcgagtc cattaccccg 300
   gagattgtcg ataaagtcta caacatcaac gttaaagggg tgatctgggg cattcaggcg 360
   gcggtcgagg cctttaagaa agagggtcac ggcgggaaaa tcatcaacgc ctgttcccag 420
   gccggccacg tcggcaaccc ggagctggcg gtatatagct cgagtaaatt cgccgtacgc 480
   ggcttaaccc agaccgccgc tcgcgacctc gcgccgctgg gcatcacagt caacggctac 540
   tgcccgggga ttgtcaaaac gccaatgtgg gccgaaattg accgccaggt gtccgaagcc 600
   gccggtaaac cgctgggtta cggtaccgcc gagttcgcca aacgcatcac cctcggccgc 660
   ctgtccgagc cggaagatgt cgccgcctgc gtctcctatc ttgccagccc ggattctgat 720
   tatatgaccg gtcagtcatt gctgatcgac ggcgggatgg tgtttaacta a 771
<210> 55
   <211> 1680
   <212> DNA
   <213> Klebsiella pneumoniae
<400> 55
   atggataaac agtatccggt tcgtcagtgg gcacatggtg cagatctggt tgttagccag     60
   ctggaagcac agggtgttcg tcagattttt ggtattccgg gtgccaaaat cgataaagtt    120
   tttgatagcc tgctggatag cagcattcgt attattccgg tgcgtcatga agcaaatgca    180
   gcatttatgg cagcagcagt tggtcgtatt accggcaaag ccggtgttgc actggttacc    240
   agcggtccgg gttgtagcaa tctgattacc ggtatggcaa ccgcaaatag cgaaggtgat    300
   ccggttgttg ccctgggtgg tgcagttaaa cgtgcagata aagcaaaaca ggttcatcag    360
   agcatggata ccgttgcaat gtttagtccg gttaccaaat atgcagttga agttaccgca    420
   ccggatgcac tggccgaagt tgttagcaat gcatttcgtg cagcagaaca gggtcgtccg    480
   ggtagcagct ttgttagcct gccgcaggat gttgttgatg gtccggttag cggtaaagtt    540
   ctgcctgcaa gccgtgcacc gcagatgggt gcagctccgg atgatgcaat tgatcaggtt    600
   gcaaaactga ttgcacaggc caaaaacccg atttttctgc tgggtctgat ggcaagccag    660
   ccggaaaata gcgcagcact gcgtcgtctg ctggaagcga gccatattcc ggttacaagc    720
   acctatcagg cagccggtgc cgttaatcag gataacttta gccgttttgc aggtcgtgtt    780
   ggtctgttta ataaccaggc aggggatcgc ctgctgcaac tggccgatct ggttatttgt    840
   attggttatt ctccggtgga atatgaaccg gcaatgtgga atagcggtaa tgcaaccctg    900
   gtgcatattg atgttctgcc agcctatgaa gaacgtaatt atacaccgga tgttgaactg    960
   gtgggtgata ttgcaggcac cctgaataaa ctggcacaga atattgatca tcgtctggtt   1020
   ctgagtccgc aggcagcaga aattctgcgt gatcgtcagc atcagcgtga actgctggat   1080
   cgtcgtggtg cacagctgaa tcagtttgca ctgcatccgc tgcgtattgt tcgtgcaatg   1140
   caggatattg ttaatagtga tgttaccctg accgttgata tgggcagctt tcatatttgg   1200
   attgcccgtt atctgtatag ctttcgtgca cgtcaggtta tgattagcaa tggtcagcag   1260
   acaatgggtg ttgcgctgcc gtgggcaatt ggtgcatggc tggttaatcc ggaacgtaaa   1320
   gttgtgagcg ttagcggtga tggtggtttt ctgcaaagca gcatggaact ggaaaccgca   1380
   gttcgtctga aagcaaatgt tctgcatctg atttgggtgg ataatggcta taatatggtg   1440
   gccatccaag aagagaaaaa ataccagcgt ctgagcggtg ttgaatttgg tccgatggat   1500
   tttaaagcct atgccgaaag ttttggtgca aaaggttttg ccgttgaaag cgcagaagca   1560
   ctggaaccga ccctgcgtgc agcaatggat gtggatggtc ctgccgttgt tgcaattccg   1620
   gttgattatc gtgataatcc gctgctgatg ggtcagctgc atctgtcaca gattctgtaa   1680
<210> 56
   <211> 930
   <212> DNA
   <213> Saccharomyces cerevisiae
<400> 56
   atgctgcgta gcctgctgca aagcggtcat cgtcgtgttg ttgcaagcag ctgtgccaca      60
   atggttcgtt gtagcagcag cagtaccagc gcactggcat ataaacaaat gcatcgtcat     120
   gcaacccgtc cgcctctgcc gaccctggat accccgagct ggaatgcaaa tagcgcagtt     180
   agcagcatta tctatgaaac accggcaccg agccgtcagc ctcgtaaaca gcatgttctg     240
   aattgtctgg ttcagaatga accgggtgtt ctgagccgtg ttagcggcac cctggcagca     300
   cgtggtttta acattgatag cctggttgtt tgcaacaccg aagttaaaga tctgagtcgt     360
   atgaccattg ttctgcaagg tcaggatggt gttgttgaac aggcacgtcg tcagattgaa     420
   gatctggttc cggtttatgc agttctggat tataccaaca gcgaaatcat taaacgtgaa     480
   ctggttatgg cacgtattag tctgctgggc accgaatatt ttgaggatct gctgctgcat     540
   catcatacca gcaccaatgc cggtgcagca gatagccaag aactggttgc agaaattcgt     600
   gaaaaacagt ttcatccggc aaatctgcct gcaagcgaag ttctgcgtct gaaacatgaa     660
   catctgaacg atattaccaa cctgaccaat aactttggtg gtcgtgtggt tgatattagc     720
   gaaaccagct gtattgttga actgagcgca aaaccgaccc gtattagcgc atttctgaaa     780
   ctggttgaac cgtttggtgt tctggaatgt gcacgtagcg gtatgatggc actgcctcgt     840
   acaccgctga aaaccagcac cgaagaggca gcagatgaag atgaaaaaat cagcgaaatt     900
   gtggatatca gccagctgcc tccgggttaa                                      930

## Description

### TECHNICAL FIELD OF THE INVENTION

The present disclosure provides non-naturally occurring microorganism having a 2,3-butanediol pathway. The recombinant microorganism is modified to improve the production of 2,3-butanediol compared to the unmodified microorganism. The disclosure also provides methods for using such microorganisms to produce 2,3-butanediol.

### BACKGROUND OF THE INVENTION

As crude oil reserves become increasingly scarce, bio-refinery systems that integrate biomass conversion processes and equipment to produce fuels, power, and chemicals from annually renewable resources are at the stage of worldwide development. Many chemicals that could only be produced by traditional chemical processes in the past can now have the potential to be generated biologically, using renewable resources (Danner & Braun, 1999; Hatti-Kaul *et al.,* 2007). Microbial production of 2,3-butanediol (2,3-BDO) is one such example. Interest in this bioprocess has increased remarkably because 2,3-BDO has a large number of industrial applications, and microbial production will alleviate the dependence on oil supply for the production of platform chemicals (Celińska & Grajek, 2009; Wu *et al.,* 2008).
2,3-BDO is also known as 2,3-butylene glycol, dimethylene glycol, dimethylethylene glycol and the IUPAC name is butane-2,3-diol. Its molecular formula is C₄H₁₀O₂. The CAS number is 513-85-9.
2,3-BDO exists in 3 isomeric forms: D-(-)-, L-(+)- and meso- as shown below:

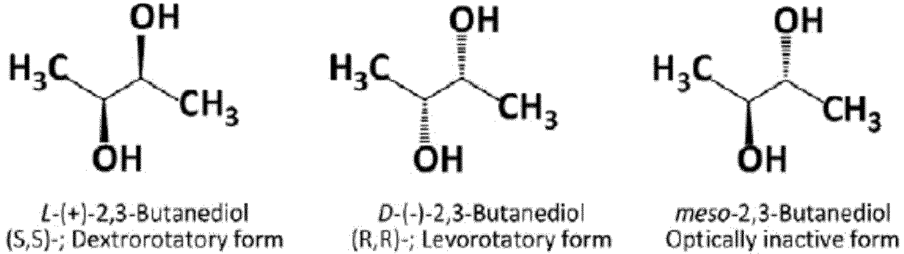

One possible application of 2,3-BDO is its conversion into 1,3-butadiene, that can be used in synthetic rubber production. Additionally, 2,3-BDO has potential applications in the manufacture of printing inks, perfumes, fumigants, moistening and softening agents, explosives, plasticizers, food, and pharmaceuticals (Garg & Jain, 1995; Syu, 2001).

Microbial 2,3-BDO production has a history of more than 100 years. It was first investigated in 1906 by Harden and Walpole and in 1912 by Harden and Norris (Magee & Kosaric, 1985). The bacterium employed in these early studies was *Klebsiella pneumoniae* (formerly *Aerobacter aerogenes* or *Klebsiella aerogenes*). Since then, the production of 2,3-BDO has been progressing and is well described in bacteria (Celińska & Grajek, 2009; Ji *et al.,* 2011), mainly risk group 2 and risk group 1 bacteria, including *E. coli* (Ui *et al.,* 2004; Nielsen *et al.,* 2010).
2,3-butanediol is a by-product of alcoholic fermentation by yeast and usually one of the most abundant minor constituent of wine. It originates from the reduction of acetoin (Romano & Suzzi, 1996). Numerous attempts have been made to engineer *Saccharomyces cerevisiae* strains with reduced acetoin yields, by re-orienting carbons toward glycerol and 2,3-BDO to obtain low-alcohol yeasts with desirable organoleptic features, permitting the decrease of the ethanol contents in wines by up to 3°C (Ehsani *et al.,* 2009; Gonzalez *et al.,* 2010).
Yeast cells have been more recently considered as a cellular mini bioreactor to produce 2,3-BDO. For instance, the patent application WO2011041426 provides yeast cells that are engineered to have improved growth and production of products, among them the 2,3-BDO. Particularly, in these yeast cells one or more pyruvate decarboxylase genes have been inactivated in order to suppress the endogenous competing pyruvate-utilizing metabolic pathway. Ehsani et al (2009) provides an example of 2,3-BDO production in *S*. *cerevisiae* overexpressing butanol dehydrogenase (BDH) with an initial conversion of pyruvate into acetaldehyde, which is then converted into acetoin. Other examples of 2,3-BDO production are described in patent application WO2010151525 and in the work of Ng *et al.* (2012).
Generally, yeast can grow rapidly and can be cultivated at higher density as compared with bacteria, and does not require an aseptic environment in the industrial setting. Furthermore, yeast cells can be more easily separated from the culture medium compared to bacterial cells, greatly simplifying the process for product extraction and purification.
The present inventors propose alternative pathways in order to improve 2,3-butanediol biosynthesis in microorganisms, including yeasts and bacteria, regarding environmental impact, performance of production, facility of recovery or economy.

The present disclosure is related to new alternative pathways for the fermentative production of 2,3-butanediol and provides non-naturally occurring microorganism having a 2,3-butanediol pathway.
In particular, the disclosure is related to a recombinant microorganism engineered to produce 2,3-butanediol (BDO) wherein said microorganism is chosen among bacterium or yeast and overexpresses at least one gene encoding a polypeptide involved in the conversion of pyruvate into 2,3-butanediol. According to the invention, 2,3-butanediol can be obtained via six different metabolic pathways.
The first pathway of the disclosure comprises genes encoding polypeptides having activity of acetolactate synthase (ALS), acetolactate decarboxylase (ALDC) and butanediol dehydrogenase (BDH).
The second pathway of the disclosure involves genes encoding a polypeptide catalysing the conversion of pyruvate to acetaldehyde, polypeptides catalysing the conversion of pyruvate to acetyl-CoA and the conversion of acetyl-CoA to acetaldehyde, a polypeptide catalysing the condensation of pyruvate and/or acetaldehyde to acetoin and a polypeptide having butanediol dehydrogenase (BDH) activity, thus reducing acetoin to 2,3-butanediol.
The third pathway of the disclosure comprises genes encoding polypeptides having activity of acetolactate synthase (ALS) and butanediol dehydrogenase (BDH).
The fourth pathway involves genes encoding polypeptides having activity of pyruvate decarboxylase (PDC) and pyruvate formate lyase (PFL), acetoin dehydrogenase (ACDH) and butanediol dehydrogenase (BDH).
The fifth pathway of the disclosure comprises genes encoding polypeptides having activity of propionate CoA transferase, beta-keto-thiolase, thioesterase, beta-keto-acid decarboxylase and butanediol dehydrogenase (BDH).
The sixth pathway of the disclosure involves genes encoding polypeptides having activity of acetolactate synthase (ALS), 3-hydroxy alkanoate decarboxylase, dehydratase, acetolactate decarboxylase and butanediol dehydrogenase (BDH).
In another aspect, the disclosure relates also to a method of production of 2,3-butanediol which comprises providing a recombinant microorganism, cultivating the recombinant microorganism in a culture medium containing a source of carbon, and recovering the 2,3-butanediol.

### SUMMARY OF THE INVENTION

The present invention is defined in claims 1 to 5.

### BRIEF DESCRIPTION OF THE DRAWINGS

Figure 1 shows the first metabolic pathway for 2,3-butanediol named Pathway 1
Figure 2 shows the second metabolic pathway for 2,3-butanediol named Pathway 2
Figure 3 shows the third metabolic pathway for 2,3-butanediol named Pathway 3
Figure 4 shows the fourth metabolic pathway for 2,3-butanediol named Pathway 4
Figure 5 shows the fifth metabolic pathway for 2,3-butanediol named Pathway 5
Figure 6 shows the sixth metabolic pathway for 2,3-butanediol named Pathway 6

### DETAILED DESCRIPTION OF THE INVENTION

Before describing the present invention in detail, it is to be understood that this invention is not limited to particularly exemplified methods and may, of course, vary. It is also to be understood that the terminology used herein is for the purpose of describing particular embodiments of the invention only, and is not intended to be limiting, which will be limited only by the appended claims.

All publications, patents and patent applications cited herein, whether *supra* or *infra,* are hereby incorporated by reference in their entirety. However, publications mentioned herein are cited for the purpose of describing and disclosing the protocols, reagents and vectors that are reported in the publications and that might be used in connection with the invention.

Furthermore, the practice of the present invention employs, unless otherwise indicated, conventional microbiological and molecular biological techniques within the skill of the art. Such techniques are well known to the skilled worker, and are explained fully in the literature. See, for example, Guthrie & Fink, 2004 and Sambrook *et al.,* (1999) (2001).

It must be noted that as used herein and in the appended claims, the singular forms "a", "an", and "the" include plural reference unless the context clearly dictates otherwise. Thus, for example, a reference to "a microorganism" includes a plurality of such microorganisms, and a reference to "an endogenous gene" is a reference to one or more endogenous genes, and so forth. Unless defined otherwise, all technical and scientific terms used herein have the same meanings as commonly understood by one of ordinary skill in the art to which this invention belongs. Although any materials and methods similar or equivalent to those described herein can be used to practice or test the present invention, the preferred materials and methods are now described.

In the claims that follow and in the preceding description of the invention, except where the context requires otherwise due to express language or necessary implication, the terms "comprise", "have", "contain" or "include" or variations such as "comprises", "comprising", "includes", "including", "has", "having", "contains" or "containing" are used in an inclusive sense, i.e. to specify the presence of the stated features but not to preclude the presence or addition of further features in various embodiments of the invention.

### Definitions

The terms 2,3-butanediol, 2,3-BDO or BDO are used interchangeably in the invention and refer to butane-2,3-diol, also called dimethylene glycol.

The term "microorganism", as used herein, refers to a bacterium, yeast or fungus which is not modified artificially. The microorganism may be "donor" if it provides genetic element to be integrated in the microorganism "acceptor" which will express this foreign genetic element or if it used as tool for genetic constructions or protein expressions.

The microorganism of the invention is chosen among bacterium, yeast or fungus which expresses genes for the biosynthesis of 2,3-butanediol.

Preferentially, the bacterium of the invention is selected among *Enterobacteriaceae, Bacillaceae, Streptomycetaceae* and *Corynebacteriaceae.* More preferentially the microorganism is a species of *Escherichia, Pantoea, Salmonella, or Corynebacterium.* Even more preferentially the microorganism is either the species *Escherichia coli* or *Corynebacterium glutamicum.*

Preferentially, the yeast of the invention is selected among the genera *Saccharomycetaceae, Candida, Kluyveromyces, Ashbya, Dekkera, Pichia* (*Hansenula*), *Debaryomyces, Clavispora, Lodderomyces, Yarrowia, Schizosaccharomyces, Cryptococcus, Malassezia.* More preferentially the yeast is a species of *Saccharomyces, Pichia, Kluyveromyces* or *Yarrowia.* According to a specific aspect of the invention, the yeast is from the species *Saccharomyces cerevisiae, Kluyveromyces lactis* or *Kluyveromyces marxianus.*

More preferentially, the bacterium is *Escherichia coli* and the yeast is *Saccharomyces cerevisiae.*

The term "recombinant microorganism" or "genetically modified microorganism" or "recombinant yeast" or "genetically modified yeast" or "recombinant bacterium" or "genetically modified bacterium", as used herein, refers to a microorganism, including yeast and bacteria, genetically modified or genetically engineered. It means, according to the usual meaning of these terms, that the microorganism of the invention is not found in nature and is modified either by introduction or by deletion or by modification of genetic elements from equivalent microorganism found in nature. It can also be transformed by forcing the development and evolution of new metabolic pathways by combining directed mutagenesis and evolution under specific selection pressure (see for instance WO2004076659).

A microorganism may be modified to express exogenous genes if these genes are introduced into the microorganism with all the elements allowing their expression in the host microorganism. A microorganism may be modified to modulate the expression level of an endogenous gene. The modification or "transformation" of microorganism like bacterium or yeast with exogenous DNA is a routine task for those skilled in the art.

The term "endogenous gene" means that the gene was present in the microorganism before any genetic modification, in the wild-type strain. Endogenous genes may be overexpressed by introducing heterologous sequences in addition to, or to replace endogenous regulatory elements, or by introducing one or more supplementary copies of the gene into the chromosome or a plasmid. Endogenous genes may also be modified to modulate their expression and/or activity. For example, mutations may be introduced into the coding sequence to modify the gene product or heterologous sequences may be introduced in addition to or to replace endogenous regulatory elements. Modulation of an endogenous gene may result in the up-regulation and/or enhancement of the activity of the gene product, or alternatively, in the down-regulation and/or attenuation of the activity of the endogenous gene product. Another way to enhance expression of endogenous genes is to introduce one or more supplementary copies of the gene onto the chromosome or a plasmid.

The term "exogenous gene" means that the gene was introduced into a microorganism, by means well known by the man skilled in the art, whereas this gene is not naturally occurring in the microorganism. Exogenous genes can be heterologous or not. Microorganism can express exogenous genes if these genes are introduced into the microorganism with all the elements allowing their expression in the host microorganism. Transforming microorganisms with exogenous DNA is a routine task for the man skilled in the art. Exogenous genes may be integrated into the host chromosome, or be expressed extra-chromosomally from plasmids or vectors. A variety of plasmids, which differ with respect to their origin of replication and their copy number in the cell, are all known in the art. These genes may be heterologous or homologous. The sequence of exogenous genes may be adapted for its expression in the host microorganism. Indeed, the man skilled in the art knows the notion of codon usage bias and how to adapt nucleic sequences for a particular codon usage bias without modifying the deduced protein.

The term "heterologous gene" means that the gene is derived from a species of microorganism different from the recipient microorganism that expresses it. It refers to a gene which is not naturally occurring in the microorganism.

In the present application, all genes are referenced with their common names and with references that give access to their nucleotidic sequences on the websites http://www.ebi.ac.uk/embl/ or http://www.ncbi.nlm.nih.gov/gene. Using the references given in Genbank for known genes, those skilled in the art are able to determine the equivalent genes in other organisms, bacterial strains, yeast, fungi, mammals, plants, etc. This routine work is advantageously done using consensus sequences that can be determined by carrying out sequence alignments with genes derived from other microorganisms and designing degenerated probes to clone the corresponding gene in another organism.

The man skilled in the art knows different means to modulate, and in particular up-regulate, the expression of endogenous genes. For example, a way to enhance expression of endogenous genes is to introduce one or more supplementary copies of the gene onto the chromosome or a plasmid.

Another way is to replace the endogenous promoter of a gene with a stronger promoter. These promoters may be homologous or heterologous.

Homologous promoters known to allow a high level of expression in yeast are the ones expressing glycolytic genes; ADH1, GPDH, TEF1, truncated HXT7, PFK1, FBA1, PGK1 and TDH3 etc... More preferably, the truncated HXT7 promoter is used in the yeasts of the invention. In yeast, nucleic acid expression construct preferably comprises regulatory sequences, such as promoter and terminator sequences, which are operatively linked with the nucleic acid sequence coding for the plant pentose transporter.

Preferred promoter sequences are HXT7, truncated HXT7, PFK1, FBA1, PGK1, ADH1 and TDH3. Preferred terminator sequences are CYC1, FBA1, PGK1, PFK1, ADH1 and TDH3. The nucleic acid expression construct may further comprise 5' and/or 3' recognition sequences and/or selection markers.

It is well within the ability of the person skilled in the art to select appropriate promoters, for example, the promoters from the collection comprising 11 mutants of the strong constitutive Saccharomyces cerevisiae TEF1 promoter are widely used (Nevoigt et al., 2007).

Homologous promoters known to allow a high level of expression in bacterium are, for instance, promoters P*trc,* P*tac,* P*lac,* or the lambda promoter *cI* which are widely used. These promoters can be "inducible" by a particular compound or by specific external condition like temperature or light. These promoters may be homologous or heterologous.

The term 'overexpression' means in this context that the expression of a gene or an enzyme is increased compared to the non-modified microorganism. Increasing the expression of an enzyme is obtained by increasing the expression of a gene encoding said enzyme.

The 'activity' of an enzyme is used interchangeably with the term 'function' and designates, in the context of the invention, the reaction that is catalyzed by the enzyme.

The terms "reduced activity" or "attenuated activity" of an enzyme mean either a reduced specific catalytic activity of the protein obtained by mutation in the amino acids sequence and/or decreased concentrations of the protein in the cell obtained by mutation of the nucleotidic sequence or by deletion of the coding gene.

The term 'enhanced activity' of an enzyme designates either an increased specific catalytic activity of the enzyme, and/or an increased quantity/availability of the enzyme in the cell, obtained for example by overexpression of the gene encoding the enzyme.

The terms "encoding" or "coding" refer to the process by which a polynucleotide, through the mechanisms of transcription and translation, produces an amino-acid sequence.

The gene(s) encoding the enzyme(s) can be exogenous or endogenous.

"Attenuation" of genes means that genes are expressed at an inferior rate than the non-modified rate. The attenuation may be achieved by means and methods known to the man skilled in the art and contains gene deletion obtained by homologous recombination, gene attenuation by insertion of an external element into the gene or gene expression under a weak promoter. The man skilled in the art knows a variety of promoters which exhibit different strengths and which promoter to use for a weak genetic expression.
The methods of the present invention require the use of one or more chromosomal integration constructs for the stable introduction of a heterologous nucleotide sequence into a specific location on a chromosome or for the functional disruption of one or more target genes in a genetically modified microbial cell. In some embodiments, disruption of the target gene prevents the expression of a functional protein. In some embodiments, disruption of the target gene results in the expression of a non-functional protein from the disrupted gene.
Parameters of chromosomal integration constructs that may be varied in the practice of the present invention include, but are not limited to, the lengths of the homologous sequences; the nucleotide sequence of the homologous sequences; the length of the integrating sequence; the nucleotide sequence of the integrating sequence; and the nucleotide sequence of the target locus. In some embodiments, an effective range for the length of each homologous sequence is 20 to 5,000 base pairs, preferentially 40 base pairs. In particular embodiments, the length of each homologous sequence is about 500 base pairs. For more information on the length of homology required for gene targeting, see Hasty *et al.,* (1991). In some embodiments, the expression vector or chromosomal integration vector used to genetically modify a microbial cell of the invention comprises one or more selectable markers useful for the selection of transformed microbial cells.
In some embodiments, the selectable marker is an antibiotic resistance marker. Illustrative examples of antibiotic resistance markers include, but are not limited to the BLA, NAT1, PAT, AUR1-C, PDR4, SMR1, CAT, mouse dhfr, HPH, DSDA, KAN^{R}, and SH BLE gene products. The BLA gene product from *E. coli* confers resistance to beta-lactam antibiotics and to all the anti-gram-negative-bacterial penicillins except temocillin; the NAT1 gene product from *S. noursei* confers resistance to nourseothricin; the PAT gene product from *S*. *viridochromogenes* Tu94 confers resistance to bialophos; the AUR1-C gene product from *Saccharomyces cerevisiae* confers resistance to Auerobasidin A (AbA); the PDR4 gene product confers resistance to cerulenin; the SMR1 gene product confers resistance to sulfometuron methyl; the CAT gene product from the Tn9 transposon confers resistance to chloramphenicol; the mouse dhfr gene product confers resistance to methotrexate; the HPH gene product of *Klebsiella pneumonia* confers resistance to Hygromycin B; the DSDA gene product of *E. coli* allows cells to grow on plates with D-serine as the sole nitrogen source; the KAN^{R} gene of the Tn903 transposon confers resistance to G418; and the SH BLE gene product from *Streptoalloteichus hindustanus* confers resistance to Zeocin (bleomycin). In some embodiments, the antibiotic resistance marker is deleted after the genetically modified microbial cell of the invention is isolated. The man skilled in the art is able to choose suitable marker in specific genetic context.
In some embodiments, the selectable marker rescues an auxotrophy (e.g., a nutritional auxotrophy) in the genetically modified microbial cell. In such embodiments, a parent microbial cell comprises a functional disruption in one or more gene products that function in an amino acid or nucleotide biosynthetic pathway, such as, for example, the HIS3, LEU2, LYS1, LYS2, MET15, TRP1, ADE2, and URA3 gene products in yeast, which renders the parent microbial cell incapable of growing in media without supplementation with one or more nutrients (auxotrophic phenotype). The auxotrophic phenotype can then be rescued by transforming the parent microbial cell with an expression vector or chromosomal integration encoding a functional copy of the disrupted gene product, and the genetically modified microbial cell generated can be selected for based on the loss of the auxotrophic phenotype of the parent microbial cell. Utilization of the URA3, TRP1, and HIS3 genes as selectable markers has a marked advantage because both positive and negative selections are possible. Positive selection is carried out by auxotrophic complementation of the URA3, TRP1, and LYS2 mutations, whereas negative selection is based on specific inhibitors, i.e., 5-fluoro-orotic acid (FOA), 5-fluoroanthranilic acid, and a-aminoadipic acid (aAA), respectively, that prevent growth of the prototrophic strains but allows growth of the URA3, TRP1, and HIS3 mutants, respectively. Preferentially, the selectable marker gene used is HIS3.
Suitable promoters, transcriptional terminators, and coding regions may be cloned into *E*. *coli*-yeast shuttle vectors, and transformed into yeast cells as described in Examples. These vectors allow propagation in both *E. coli* and yeast strains. The vector contains a selectable marker and sequences allowing autonomous replication, or chromosomal integration in the desired host. Used plasmids in yeast are for example shuttle vectors p423H7, p424H7,p425H7 et p426H7 (Wieczorke *et al.,* 1999 and Hamacher *et al.,* 2002) which contain a *E. coli* replication origin, a yeast origin of replication, and a marker for nutritional selection. The selection markers for these four vectors are HIS3, TRP1, LEU2 and URA3.
In other embodiments, the selectable marker rescues other non-lethal deficiencies or phenotypes that can be identified by a known selection method.

The "fermentation" or "culture" is generally conducted in fermenters with an appropriate culture medium adapted to the microorganism being cultivated, containing at least one simple carbon source, and if necessary co-substrates.
Microorganisms disclosed herein may be grown in fermentation media for the production of a product from pyruvate. For maximal production of some products such as 2,3-butanediol, the microorganism strains used as production hosts preferably have enhanced tolerance to the produced chemical, and have a high rate of carbohydrate utilization. These characteristics may be conferred by mutagenesis and selection, genetic engineering, or may be natural.
Fermentation media, or 'culture medium', for the present cells contain at least about 2 g/L glucose. Additional carbon substrates may include but are not limited to monosaccharides such as fructose, mannose, xylose and arabinose, oligosaccharides such as lactose maltose, galactose, or sucrose, polysaccharides such as starch or cellulose or mixtures thereof and unpurified mixtures from renewable feedstocks such as cheese whey permeate cornsteep liquor, sugar beet molasses, and barley malt. Other carbon substrates may include ethanol, lactate, succinate, or glycerol. Hence it is contemplated that the source of carbon utilized in the present invention may encompass a wide variety of carbon containing substrates and will only be limited by the choice of organism. In a specific embodiment of the invention, the substrate may be hydrolysate of second generation carbons sources such wheat straw, miscanthus plants, sugar cane bagasses, wood and others that are known to the man skilled in the art.
Although it is contemplated that all of the above mentioned carbon substrates and mixtures thereof are suitable in the present invention, preferred carbon substrates are glucose, fructose, and sucrose, or mixtures of these with C5 sugars such as xylose and/or arabinose for microorganisms modified to use C5 sugars.
In addition to an appropriate carbon source, fermentation media must contain suitable minerals, salts, cofactors, buffers and other components, known to those skilled in the art, suitable for the growth of the cultures and promotion of the enzymatic pathway necessary for the production of the desired product.

The terms "anaerobic conditions" refer to conditions under which the oxygen concentration in the culture medium is too low for the microorganism to be used as terminal electron acceptor. Anaerobic conditions may be achieved either by sparging the culture medium with an inert gas such as nitrogen until oxygen is no longer available to the microorganism or by the consumption of the available oxygen by the microorganism.

"Aerobic conditions" refers to concentrations of oxygen in the culture medium that are sufficient for an aerobic or facultative anaerobic microorganism to use as a terminal electron acceptor.

"Microaerobic condition" refers to a culture medium in which the concentration of oxygen is less than that in air, i.e; oxygen concentration up to 6% O₂.

An "appropriate culture medium" designates a medium (e.g., a sterile, liquid media) comprising nutrients essential or beneficial to the maintenance and/or growth of the cell such as carbon sources or carbon substrate, nitrogen sources, for example, peptone, yeast extracts, meat extracts, malt extracts, urea, ammonium sulfate, ammonium chloride, ammonium nitrate and ammonium phosphate; phosphorus sources, for example, monopotassium phosphate or dipotassium phosphate; trace elements (e.g., metal salts), for example magnesium salts, cobalt salts and/or manganese salts; as well as growth factors such as amino acids, vitamins, growth promoters, and the like.

The term "carbon source" or "carbon substrate" or "source of carbon" according to the present invention denotes any source of carbon that can be used by those skilled in the art to support the normal growth of a microorganism, including hexoses (such as glucose, galactose or lactose), pentoses, monosaccharides, oligosaccharides, disaccharides (such as sucrose, cellobiose or maltose), molasses, starch or its derivatives, cellulose, hemicelluloses and combinations thereof.

### 2,3-butanediol biosynthesis pathways

The present disclosure is related to a recombinant microorganism engineered to express a 2,3-butanediol pathway, by overexpressing at least one gene encoding a polypeptide involved in the conversion of pyruvate to 2,3-butanediol.

Preferentially, the recombinant microorganism disclosed herein overexpresses at least one exogenous gene involved in the 2,3-butanediol biosynthesis.

In one aspect of the disclosure, the recombinant microorganism exhibits a reduced production of 2,3-butanediol by-products.

In yeast, the main 2,3-butanediol by-product is ethanol. Thus, activity of at least one enzyme catalysing the conversion of acetaldehyde to ethanol chosen among ADH1, ADH2, ADH3, ADH4, ADH5, ADH6, ADH7 and SFA1 is reduced.
Yeast is known as a very efficient ethanol producer during fermentation. Ethanol is produced from pyruvate by an enzyme having alcohol dehydrogenase (ADH) activity. Eight different ADH are present in yeast: ADH1, ADH2, ADH3, ADH4, ADH5, ADH6, ADH7 and SFA1 encoded respectively by *ADH1, ADH2, ADH3, ADH4, ADH5, ADH6, ADH7* and *SFA1* genes (Smidt *et al.,* 2012). To increase the productivity of 2,3-butanediol, it is necessary to decrease the ethanol production.

Preferentially, the recombinant yeast cell exhibits a reduced activity of at least one enzyme catalysing the conversion of acetaldehyde to ethanol, chosen among: ADH1, ADH2, ADH3, ADH4, ADH5, ADH6, ADH7 and SFA1.

In particular, at least the activity of ADH1 is reduced. For example, the activities of ADH1, ADH2, ADH3, ADH4, ADH5 and SOFA are reduced in the recombinant yeast cell. More preferably, the activities of ADH1, ADH3, ADH5 are reduced in the recombinant yeast cell. Preferably, ADH activities are reduced by attenuating expression of the corresponding coding genes. In particular, according to the disclosure, the *ADH1* gene is deleted in the recombinant yeast cell. More preferably, the *ADH1, ADH2, ADH3, ADH4, ADH5* and *SFA1* genes are deleted. Even more preferably, the *ADH1, ADH3* and *ADH5* genes are deleted.

In bacterium, the main 2,3-butanediol by-products are lactate and acetate. Thus, activity of at least one enzyme catalysing the conversion of pyruvate to lactate or acetate is reduced. Preferably, activity of at least one enzyme chosen among LdhA, PoxB, AckA, Pta or AceEF is reduced. More preferably, activities of all LdhA, PoxB, AckA and Pta are reduced.

Preferably, such activities are reduced by attenuating expression of the corresponding coding genes. In particular, expression of at least one gene chosen among *ldhA, poxB, ackA, pta* or *aceEF* is attenuated. More particularly, all genes *ldhA, poxB, ackA* and *pta* are deleted.

According to the disclosure, the 2,3-butanediol is produced in a recombinant microorganism via six different metabolic pathways, that are described below.

### Pathway 1

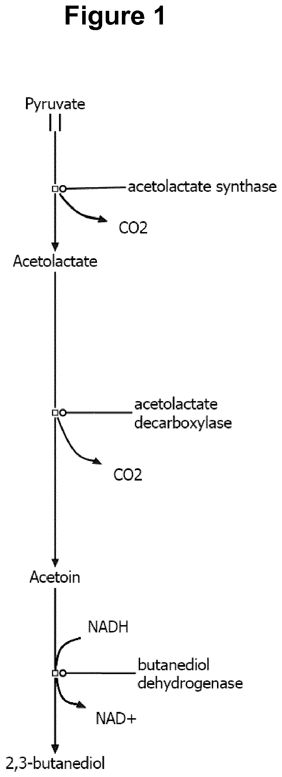

The first pathway of 2,3-butanediol biosynthesis (Figure 1) includes the following successive steps of : a) conversion of pyruvate to acetolactate by acetolactate synthase (ALS), b) conversion of acetolactate to acetoin by acetolactate decarboxylase (ALDC) and c) conversion of acetoin to 2,3-butanediol by butanediol dehydrogenase (BDH).

In an aspect of the disclosure, the recombinant microorganism expresses genes encoding polypeptides having an activity of acetolactate synthase (ALS), acetolactate decarboxylase (ALDC) and butanediol dehydrogenase (BDH).

The different genes are endogenous genes or exogenous genes. The man skilled in the art knows that polypeptide having ALS, ALDC or BDH activity isolated from a variety of organisms may be used in the present recombinant microorganism. Identification of similar nucleotides or amino acids sequences using bioinformatics algorithms such as BLAST (Altschul *et al.,* 1993) on publicly available databases with known sequences is well done by the skilled person.

ALS enzymes which may also be called acetohydroxy acid synthases and belong to the class Enzyme Class 2.2.1.6 are well known by the man skilled in the art, as they participate in the biosynthetic pathway for the proteinogenic amino acids leucine and valine and in the fermentative production of 2,3-butanediol in many organisms.

In the present disclosure, ALS enzymes that have substrate specificity for pyruvate over ketopyruvate are preferred.

Enzymes having ALS activity are encoded by one of the following genes: *ilvB* and *ilvN* from *Corynebacterium glutamicum, ilvB* and *ilvN* from *E. coli, budB* and *alsS* from *Klebsiella pneumoniae* 342, *budB* from *Klebsiella oxytoca, budB* from *Enterobacter aerogenes, slaB* from *Serratia marcescens, ILV2* and *ILV6* from *Saccharomyces cerevisiae, alsS* from *Bacillus subtilis subtilis* 168, *alsS* from *Bacillus thuriengensis* or *PPSC2_c1472* from *Bacillus polymyxa.* Any ALS coding gene having at least 80-85%, preferentially 85-90%, more preferentially 90-95%, even more preferentially 96%, 97%, 98%, 99% sequence identity to any of those genes may be used.

For example, the expressed ALS enzyme is encoded by the gene *budB* from *Klebsiella pneumoniae.*

In another example, the expressed ALS enzyme is encoded by the genes *ILV2* or *ILV6* from *Saccharomyces cerevisiae.*

More preferentially, in the bacterium disclosed herein ALS enzyme is encoded by the gene *budB* from *Klebsiella pneumoniae* whereas in the yeast of the disclosure ALS enzyme is encoded by the gene *ILV2* from *Saccharomyces cerevisiae.*

In order to optimize 2,3-butanediol biosynthesis in yeast the ALS is specifically expressed in the cytosol. This cytosolic expression is achieved by transforming the yeast with a gene encoding an ALS lacking the mitochondrial targeting signal sequence as described in Brat *et al.* (2012).

ALDC enzymes belonging to Enzyme Class 4.1.1.5 and being naturally absent from yeast are encoded by the genes *budA* from *Klebsiella pneumoniae, budA* from *Klebsiella oxytoca, budA* from *Enterobacter aerogenes, salA* from *Serratia marcescens, alsD* from *Bacillus subtilis subtilis* 168, *alsD* from *Bacillus thuriengiensis* or *PPSC2_c2281* from *Bacillus polymyxa.* Any ALDC encoding gene having at least 80-85%, preferentially 85-90%, more preferentially 90-95%, even more preferentially 96%, 97%, 98%, 99% sequence identity to any of those genes may be used.

For example, the expressed ALDC enzyme is encoded by the gene *budA* from *Klebsiella pneumoniae* in the bacterium and the yeast disclosed herein.

BDH enzymes may also be called acetoin reductase. BDH enzymes may have specificity for the production of (R)-2,3-butanediol (EC 1.1.1.4) or (S)-2,3-butanediol (EC 1.1.1.76). If one of the two forms is preferred, the man skilled in the art will know which enzyme to choose and to overproduce in the recombinant cell to promote the specific production.

Enzymes having BDH activity are in particular encoded by one of the following genes: *butA* from *Corynebacterium glutamicum, budC* from *Klebsiella pneumoniae, budC* from *Klebsiella oxytoca, EAE*_*17490* from *Enterobacter aerogenes, slaC* from *Serratia marcescens, BDH1* and *BDH2* from *Saccharomyces cerevisiae, bdhA* from *Bacillus subtilis subtilis* 168, *BMB171_C0587* from *Bacillus thuriengensis* or *PPSC2*_*c3335* and *PPSC2_c3890* from *Bacillus polymyxa.* Any BDH coding gene having at least 80-85%, preferentially 85-90%, more preferentially 90-95%, even more preferentially 96%, 97%, 98%, 99% sequence identity to any of those genes may be used.

For example, the expressed BDH enzyme is encoded by the gene *budC* from *Klebsiella pneumoniae.*

As another example, the expressed BDH enzyme is encoded by the genes *BDH1* and *BDH2* from *Saccharomyces cerevisiae.*

More preferentially, in the bacterium disclosed herein, BDH enzyme is encoded by the gene *budC* from *Klebsiella pneumoniae* whereas in the yeast disclosed herein, BDH enzyme is encoded by the gene *BDH1* from *Saccharomyces cerevisiae* or BDH2 from *Saccharomyces cerevisiae.*

For example, the recombinant bacterium disclosed herein expresses genes *budB, budA* and *budC* from *Klebsiella pneumoniae* ,whereas endogenous genes encoding ALS, ALDC and BDH are deleted.

In another example, the recombinant yeast disclosed herein expresses the *budA* gene from *Klebsiella pneumoniae, ILV2* and *BDH1* genes from *Saccharomyces cerevisiae.* Preferably, the recombinant yeast cell exhibits reduced activity of at least one enzyme catalysing the conversion of pyruvate to acetaldehyde in way to improve the conversion of pyruvate to acetolactate. Preferentially the expression of at least one gene chosen among *PDC1*, *PDC5* or *PDC6* is attenuated in the yeast of the disclosure. More preferentially the *PDC1* gene is deleted. More preferentially the genes *PDC1, PDC5* and *PDC6* are deleted in the modified yeast.

### Pathway 2

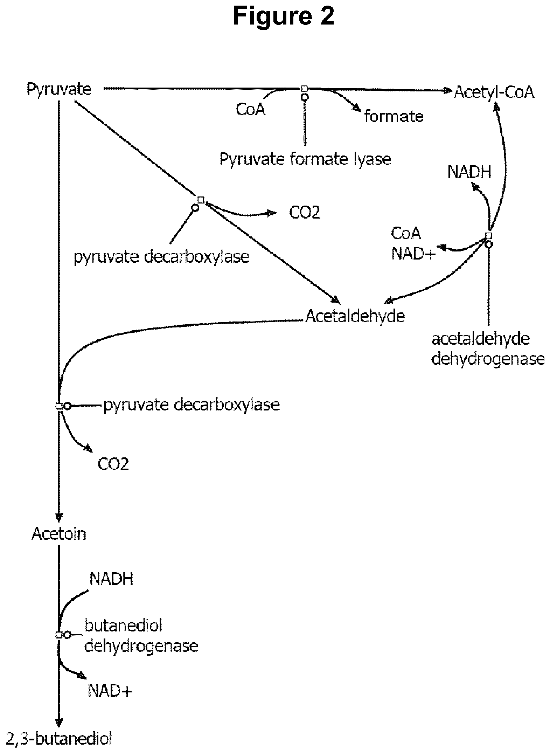

The second pathway of 2,3-butanediol biosynthesis (Figure 2) includes the successive steps of conversion of pyruvate to acetaldehyde and/or to acetyl-CoA, conversion of acetyl-CoA to acetaldehyde, conversion of pyruvate and/or acetaldehyde to acetoin and conversion of acetoin to 2,3-butanediol by butanediol dehydrogenase (BDH).

In this aspect of the disclosure, the terms "conversion of pyruvate and/or acetaldehyde to acetoin" refers either to the conversion of one molecule of pyruvate and one molecule of acetaldehyde into acetoin, or to the condensation of two molecules of acetaldehyde into acetoin.

The recombinant microorganism expresses the following enzymes:
a) A polypeptide catalysing the conversion of pyruvate to acetaldehyde or two polypeptides, one catalysing the conversion of pyruvate to acetyl-CoA and the other catalysing the conversion of acetyl-CoA to acetaldehyde, and
b) a polypeptide catalysing the condensation of pyruvate and acetaldehyde to acetoin or a polypeptide catalysing the condensation of acetaldehyde to acetoin, and
c) a butanediol dehydrogenase.

In one aspect, the recombinant microorganism expresses genes encoding polypeptides having activity of conversion of pyruvate to acetaldehyde, conversion of pyruvate and/or acetaldehyde to acetoin and butanediol dehydrogenase (BDH).

The conversion of pyruvate to acetaldehyde and of pyruvate and/or acetaldehyde to acetoin may be achieved by the same enzyme: the pyruvate decarboxylase (PDC). PDC enzyme exhibits several activities: the major activity is the conversion of pyruvate to acetaldehyde and its secondary activity is the conversion of pyruvate and/or acetaldehyde to acetoin. The secondary activity of PDC allows the conversion of pyruvate and acetaldehyde to acetoin or the condensation of two acetaldehydes to acetoin (Romano & Suzzi, 1996). This second activity is named "acetoin synthase".

For example, at least one of the *PDC1, PDC5, PDC6* genes of *Saccharomyces cerevisiae* are overexpressed in the recombinant bacterium disclosed herein. More preferentially, PDC1 from *Saccharomyces cerevisiae* is overexpressed in the recombinant bacterium.

In another example, the PDC enzyme may be evolved to enhance its acetoin synthase activity. The evolved enzymes are screened on their performance of conversion of pyruvate and/or acetaldehyde to acetoin. A preferred evolved PDC is a PDC which exhibit an improved activity of acetoin synthase of at least 20% compared to the non-evolved PDC. Several PDC mutants are disclosed in Liu *et al.* (2011). Preferably, PDC mutant Glu477Gln is used.

In another example, the PDC enzymes may be evolved to reduce its pyruvate decarboxylase activity, while keeping its acetoin synthase activity constant or increasing its acetoin synthase activity. In a further example, the ratio of acetoin synthase over pyruvate decarboxylase activity needs to be improved by at least a factor two, preferentially by a factor of ten and most preferred by a factor of 100, while increasing the acetoin synthase activity of the enzyme.

In another example, the acetoin synthase activity of PDC is replaced by an evolved glyoxylate carboxy-lyase enzyme (EC 4.4.1.47) or a novel enzyme exhibiting the same activity of conversion of pyruvate and/or acetaldehyde to acetoin. The evolved enzymes are obtained by evolution of an enzyme having carboxy-lyase activity and are screened on their specificity for their substrate. For example, the natural glyoxylate carboxy-lyase, encoded by the *gcl* gene from *Escherichia coli,* has substrate specificity for glyoxylate whereas the evolved glyoxylate carboxy-lyase presents substrate specificity for pyruvate and/or acetaldehyde.

In another example, the acetaldehyde may be obtained by conversion of acetyl-CoA by acetaldehyde dehydrogenase. The acetaldehyde dehydrogenase is encoded for example by the *mhpF* gene from *Escherichia coli K12,* the *dmpF* gene from *Pseudomonas sp* or *eutE* from *Salmonella enterica* the acetaldehyde dehydrogenase activity residing in the N-terminus of *E. coli adhE.* Preferably the microorganism of the disclosure overexpresses *mhpF* gene from *E. coli.*

The used acetyl-CoA is obtained from pyruvate by pyruvate formate lyase activity which leads to the production of acetyl-CoA and formate. In this context, the recombinant microorganism will overexpress the *pflB* and/or *pflA* genes from *Escherichia coli* or any other pyruvate formate lyase enzyme known to the expert in the art.

In a further example, formate may be recycled in the cell by overexpressing enzymatic complexes like FDH1 from *Saccharomyces cerevisiae* (Van Den Berg & Steensma 1997), FDH2 from *Saccharomyces cerevisiae* (Overkamp *et al.* 2002), *fdhl* of *Candida boidinii* (Lu *et al.* 2009), formate hydrogen lyase complex (FHL) from *Escherichia coli* (Sawers 2005).

As above, enzymes having BDH activity are encoded by genes: *butA* from *Corynebacterium glutamicum, budC* from *Klebsiella pneumoniae* 342, *budC* from *Klebsiella oxytoca, EAE*_*17490* from *Enterobacter aerogenes, slaC* from *Serratia marcescens, BDH1* and *BDH2* from *Saccharomyces cerevisiae, bdhA* from *Bacillus subtilis subtilis* 168, *BMB171_C0587* from *Bacillus thuriengensis* or *PPSC2_c3335* and *PPSC2_c3890* from *Bacillus polymyxa.* Any BDH coding gene having at least 80-85%, preferentially 85-90%, more preferentially 90-95%, even more preferentially 96%, 97%, 98%, 99% sequence identity to any of those genes may be used.

For example, the BDH used is encoded by the gene *budC* from *Klebsiella pneumoniae.*

For example, the expressed BDH enzyme is encoded by the genes *BDH1* and/or *BDH2* from *Saccharomyces cerevisiae.*

More preferentially, in the bacterium disclosed herein, BDH enzyme is encoded by the gene *budC* from *Klebsiella pneumoniae* whereas in the yeast disclosed herein, BDH enzyme is encoded by the gene *BDH1* or BDH2 from *Saccharomyces cerevisiae.*

More specifically, the present invention relates to microorganisms comprising the modifications defined in claims 1 to 4.
In wild type cells, in anaerobiosis, reductive power production by substrate catalysis is balanced with synthesis of reduced molecules, such as ethanol or acetate. In the present recombinant microorganisms, ethanol and acetate production pathways are disrupted, leading to an unbalanced redox state. Any method allowing an increase of NADH-dependent enzyme activity in the present production host cell may be used in balancing redox. For instance, addition of the pyruvate formate lyase PFLAB in absence of oxygen, allows the utilization of one more NADH during the conversion of acetyl-CoA into acetaldehyde and achieves the production of 2,3-BDO.

In a particular example, the recombinant bacterium disclosed herein overexpresses *PDC1* gene from *Saccharomyces cerevisiae, pflB* and *mhpF* genes from *Escherichia coli,* and *budC* gene from *Klebsiella pneumoniae,* whereas endogenous genes encoding ALS, ALDC and BDH and *poxB, ldhA, ackA* and *pta* genes are deleted.

In another particular example, the recombinant yeast disclosed herein expresses the *PDC1* gene from *Saccharomyces cerevisiae* and overexpressed *pflA, pflB* and *mhpF* genes from *Escherichia coli,* and *BDH1* gene from *Saccharomyces cerevisiae,* whereas *ADH1, ADH3, ADH5* and *ILV2* endogenous genes from *Saccharomyces cerevisiae* are deleted.

### Pathway 3

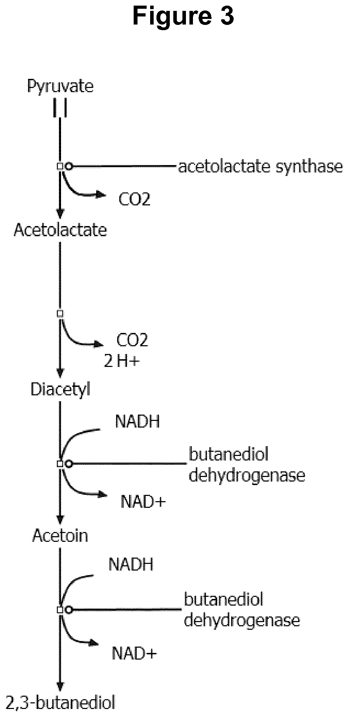

The third pathway of 2,3-butanediol biosynthesis (Figure 3) includes steps of conversion of pyruvate to acetolactate by acetolactate synthase (ALS), conversion of acetolactate to diacetyl, conversion of diacetyl to acetoin and then acetoin to 2,3-butanediol by butanediol dehydrogenase (BDH).

In one example, the recombinant microorganism disclosed herein expresses genes encoding polypeptides having activity of acetolactate synthase (ALS) and butanediol dehydrogenase (BDH).

As seen above, enzymes having ALS activity are encoded by genes: *ilvB* and *ilvN* from *Corynebacterium glutamicum, ilvB* and *ilvN* from *Escherichia coli, budB* and *alsS* from *Klebsiella pneumoniae* 342, *budB* from *Klebsiella oxytoca, budB* from *Enterobacter aerogenes, slaB* from *Serratia marcescens, ILV2* and *ILV6* from *Saccharomyces cerevisiae, alsS* from *Bacillus subtilis subtilis* 168, *alsS* from *Bacillus thuriengensis* or *PPSC2*_*c1472* from *Bacillus polymyxa.* Any ALS coding gene having at least 80-85%, preferentially 85-90%, more preferentially 90-95%, even more preferentially 96%, 97%, 98%, 99% sequence identity to any of those genes may be used.

For example, the expressed ALS enzyme is encoded by the gene *budB* from *Klebsiella pneumoniae.*

In another example, the expressed ALS enzyme is encoded by the genes *ILV2* and *ILV6* from *Saccharomyces cerevisiae.*

More preferentially, in the bacterium disclosed herein, ALS enzyme is encoded by the gene *budB* from *Klebsiella pneumoniae* whereas in the yeast disclosed herein, ALS enzyme is encoded by the gene *ILV2* from *Saccharomyces cerevisiae.*

In order to optimize 2,3-butanediol biosynthesis in yeast the ALS is specifically expressed in the cytosol. This cytosolic expression is achieved by transforming the yeast with a gene encoding an ALS lacking the mitochondrial targeting signal sequence as described in Brat *et al.* (2012).

Enzymes having BDH activity are encoded by genes: *butA* from *Corynebacterium glutamicum, budC* from *Klebsiella pneumoniae* 342, *budC* from *Klebsiella oxytoca, EAE_17490* from *Enterobacter aerogenes, slaC* from *Serratia marcescens, bdh1* and *bdh2* from *Saccharomyces cerevisiae, bdhA* from *Bacillus subtilis subtilis* 168, *BMB171_C0587* from *Bacillus thuriengensis* or *PPSC2_c3335* and *PPSC2_c3890* from *Bacillus polymyxa.* Any BDH coding gene having at least 80-85%, preferentially 85-90%, more preferentially 90-95%, even more preferentially 96%, 97%, 98%, 99% sequence identity to any of those genes may be used.

For example, the BDH used is encoded by the gene *budC* from *Klebsiella pneumoniae.*

As another example, the expressed BDH enzyme is encoded by the genes *BDH1* and/or *BDH2* from *Saccharomyces cerevisiae.*

More preferentially, in the bacterium disclosed herein, BDH enzyme is encoded by the gene *budC* from *Klebsiella pneumoniae* whereas in the yeast disclosed herein, BDH enzyme is encoded by the gene *BDH1* from *Saccharomyces cerevisiae.*
In wild-type microorganism, conversion of acetolactate to diacetyl occurs spontaneously but slowly in presence of oxygen (Bassit *et al.,* 1993).

In order to improve this conversion, gene encoding enzyme catalysing such conversion like *ILV6* from *S. cerevisiae* or *loxL2* from *Lactobacillus sakei* are overexpressed in the microorganisms disclosed herein. The lactate:oxygen 2-oxidoreductase encoded by *loxL2* gene is evolved to obtain an enzyme having activity of conversion of acetolactate to diacetyl. The evolved enzymes are screened for their substrate specificity on acetolactate.

The 2,3-butanediol production pathway can be fully balanced under anaerobic conditions, however for the production of diacetyl oxygen is needed. Thus this problem can be solved by inactivating the respiratory chain to avoid the oxidation of NADH by aerobic respiration and to guarantee complete reduction of diacetyl to 2,3-butanediol, even in presence of oxygen.
It has been shown that in wild type *Escherichia coli,* the fermentation phenotype in presence of oxygen can be obtain by deleting the main cytochrome oxydase (cytochrome bo, bd-I and bd-II) (Portnoy *et al.,* 2010).

In one example, the cytochrome C oxydase of the yeast can be disrupted in order to obtain a fermentation phenotype despite the presence of oxygen. More precisely, we knock out one or several mitochondrial genes among COX1, COX2 and COX3. In combination with other modifications previously described this will promote the production of 2,3-butanediol via diacetyl, the sole fermentative pathway allowing redox balance.

In another aspect, the present disclosure relates to a method for producing 2,3-butanediol comprising the following steps: Step 1) preparing a strain modified with pathway 3 producing acetolactate, the precursor of the diacetyl by fermentation of the strain; Step 2) spontaneous decarboxylation of acetolactate in presence of oxygen by breaking the cells, for production of the diacetyl precursor of acetoin; Step 3) production of the 2,3-butanediol by enzymatic reaction process with the diacetyl precursor as a substrate and the converting enzyme 2,3-butanediol dehydrogenase.
In step 3, the bioconversion reaction transforms the diacetyl recovered from step 2 to acetoin, which is in turn enzymatically converted to 2,3-butanediol by the BDH enzyme overproduced in a different microorganism strain which is cultivated separately.

As an example, the recombinant bacterium disclosed herein expresses *alsS* and *budC* from *Klebsiella pneumoniae* and *ILV6* gene from *Saccharomyces cerevisiae,* whereas endogenous genes encoding ALS, ALDC and BDH and *poxB, ldhA, ackA* and *pta* genes are deleted. Moreover, in this recombinant bacterium, expressions of aceE and aceF genes are attenuated.

As another example, the recombinant yeast disclosed herein expresses the *ILV2* and *BDH1* genes from *Saccharomyces cerevisiae* whereas *ADH1*, *ADH3* and *ADH5* endogenous genes from *Saccharomyces cerevisiae* are deleted.

Moreover, the recombinant yeast cell exhibits reduced activity of at least one enzyme catalysing the conversion of pyruvate to acetaldehyde in way to improve the conversion of pyruvate to acetolactate. Preferentially at least one gene chosen among *PDC1*, *PDC5* or *PDC6* is attenuated in the yeast disclosed herein. More preferentially the PDC1 gene is deleted. More preferentially the genes *PDC1*, *PDC5* and *PDC6* are deleted in the modified yeast.

### Pathway 4

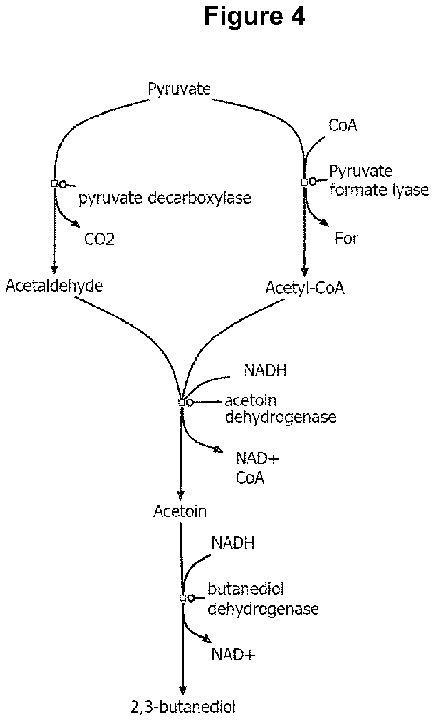

The fourth pathway of 2,3-butanediol biosynthesis (Figure 4) includes steps of a) conversion of pyruvate to acetaldehyde by pyruvate decarboxylase (PDC) and to acetyl-CoA by pyruvate formate lyase (PFL), b) condensation of acetaldehyde and acetyl-CoA to acetoin by an acetoin dehydrogenase and c) conversion of acetoin to 2,3-butanediol by butanediol dehydrogenase (BDH).

The recombinant microorganism expresses the following enzymes:
a) a polypeptide catalysing the conversion of pyruvate to acetaldehyde and a polypeptide catalysing the conversion of pyruvate to acetyl-CoA, and
b) a polypeptide catalysing the condensation of acetyl-CoA and acetaldehyde to acetoin, and
c) a butanediol dehydrogenase (BDH).

As an example, the recombinant microorganism disclosed herein expresses genes encoding polypeptides having activity of pyruvate decarboxylase (PDC), pyruvate formate lyase (PFL), acetoin dehydrogenase and butanediol dehydrogenase (BDH).

As described above, in yeast the PDC enzymes are encoded by the genes *PDC1*, *PDC5* and *PDC6.* As an example, at least one of the *PDC1, PDC5, PDC6* genes of *Saccharomyces cerevisiae* are overexpressed in the recombinant bacterium disclosed herein. More preferentially, PDC1 from *Saccharomyces cerevisiae* is overexpressed in the recombinant bacterium.

In another example, the recombinant yeast overexpresses the *pflA* and/or *pflB* genes of *Escherichia coli.* As in pathway 2 formate may be purified or disintegrated.

Enzymes having BDH activity are encoded by genes: *butA* from *Corynebacterium glutamicum, budC* from *Klebsiella pneumoniae* 342, *budC* from *Klebsiella oxytoca, EAE_17490* from *Enterobacter aerogenes, slaC* from *Serratia marcescens, BDH1* and *BDH2* from *Saccharomyces cerevisiae, bdhA* from *Bacillus subtilis subtilis* 168, *BMB171_C0587* from *Bacillus thuriengensis* or *PPSC2*_*c3335* and *PPSC2*_*c3890* from *Bacillus polymyxa.* Any BDH coding gene having at least 80-85%, preferentially 85-90%, more preferentially 90-95%, even more preferentially 96%, 97%, 98%, 99% sequence identity to any of those genes may be used.

As an example, the BDH used is encoded by the gene *budC* from *Klebsiella pneumoniae.*

In another example, the expressed BDH enzyme is encoded by the genes *BDH1* and *BDH2* from *Saccharomyces cerevisiae.*

More preferentially, in the bacterium disclosed herein, BDH enzyme is encoded by the gene *budC* from *Klebsiella pneumoniae* whereas in the yeast disclosed herein, BDH enzyme is encoded by the gene *BDH1* from *Saccharomyces cerevisiae.*

Acetoin dehydrogenase activity is catalysed for example by the protein AcoA and AcoB encoded by genes *acoA* and *acoB* from *Bacillus subtilis,* AcoB encoded by the gene *acoB* from *Pseudomonas putida,* ButA encoded by the gene *butA* from *Streptococcus gordonii,* AcoA, AcoB and AcoC encoded by genes *acoA, acoB* and *acoC* from *Pelobacter carbinolicus* or by AcoC encoded by the gene *acoC* from *Bacillus licheniformis.* The different proteins or genes may be modified in order to improve the activity of condensation of acetaldehyde and acetyl-CoA to acetoin. Preferably, *acoA* and *acoB* from *Bacillus subtilis* are overexpressed in the microorganism disclosed herein.

As an example, the recombinant bacterium disclosed herein overexpresses *PDC1* gene from *Saccharomyces cerevisiae, pflB* gene from *Escherichia coli, acoA* and *acoB* genes from *Bacillus subtilis* and *budC* gene from *Klebsiella pneumoniae,* whereas endogenous genes encoding ALS, ALDC and BDH and *poxB, ldhA, ackA* and *pta* genes are deleted.

In another example, the recombinant yeast disclosed herein expresses the PDC1 gene from *Saccharomyces cerevisiae* and overexpressed *pflA* and *pflB* genes from *Escherichia coli, acoA* and *acoB* genes from *Bacillus subtilis* and *BDH1* gene from *Saccharomyces cerevisiae,* whereas *ADH1*, *ADH3, ADH5* and *ILV2* endogenous genes from *Saccharomyces cerevisiae* are deleted.

### Pathway 5

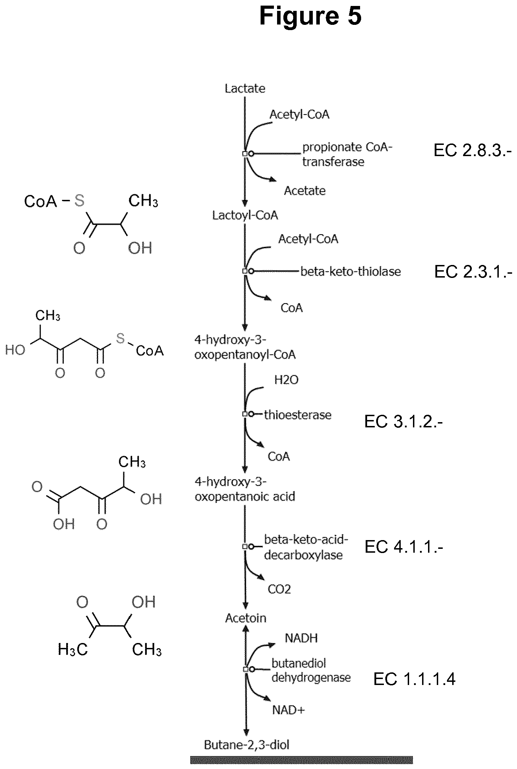

The fifth pathway of 2,3-butanediol biosynthesis (Figure 5) includes steps of conversion of lactate to lactoyl-CoA by propionate-CoA transferase, conversion of lactoyl-CoA to 4-hydroxy-3-oxopentanoyl-CoA by beta-keto-thiolase, conversion of 4-hydroxy-3-oxopentanoyl-CoA to 4-hydroxy-3-oxopentanoic acid by thioesterase, conversion of 4-hydroxy-3-oxopentanoic acid to acetoin by beta-keto-acid-decarboxylase and conversion of acetoin to 2,3-butanediol by butanediol dehydrogenase (BDH).

In this pathway, the lactate is obtained by conversion of pyruvate into lactate by the lactate dehydrogenase activity.

As an example, the recombinant microorganism disclosed herein expresses genes encoding polypeptides having activity of propionate-CoA transferase, beta-keto-thiolase, thioesterase, beta-keto-acid-decarboxylase and butanediol dehydrogenase (BDH).

Enzymes having propionate-CoA transferase activity are for example enzyme encoded by the *pct* gene from *Listeria monocytogenes_F2365,* the *pct* gene from *Wisteria welshimeri,* the *pct* gene from *Roseobacter denitrificans,* the *pct* gene from *Ralstonia eutropha,* the *pct* gene from *Rhizobium leguminosarum* or the *ydiF* gene from *Escherichia coli_APEC.*

Enzyme having beta-keto-thiolase activity are for example enzyme encoded by the *paaJ* gene from *Escherichia coli,* the *pcaF* gene from *Pseudomonas knackmussii,* the *phaD* or *pcaF* gene from *Pseudomonas putida* or the *paaE* from *Pseudomonas fluorescens.*

Enzymes having thioesterase activity are for example enzyme encoded by the *ACOT12* gene from *Bos taurus,* the *yneP* gene from *Bacillus subtilis,* the *ypch01226* gene from *Rhizobium etli,* the *hibch* gene from *Mus musculus,* the *hibch* gene from *Ratus norvegicus.*

Enzymes having beta-keto-acid-decarboxylase activity are for example enzyme coded by the *kivD* gene from *Lactococcus lactis,* the *pdc* gene from *Clostridium acetobutylicum* or by the *pdc2* gene from *Pichia stipitis.*

Enzymes having BDH activity are encoded by genes: *butA* from *Corynebacterium glutamicum, budC* from *Klebsiella pneumoniae* 342, *budC* from *Klebsiella oxytoca, EAE_17490* from *Enterobacter aerogenes, slaC* from *Serratia marcescens, BDH1* and *BDH2* from *Saccharomyces cerevisiae, bdhA* from *Bacillus subtilis subtilis* 168, *BMB171_C0587* from *Bacillus thuriengensis* or *PPSC2*_*c3335* and *PPSC2_c3890* from *Bacillus polymyxa.*

Any protein encoding a gene having at least 80-85%, preferentially 85-90%, more preferentially 90-95%, even more preferentially 96%, 97%, 98%, 99% sequence identity to any of those genes may be used.

### Pathway 6

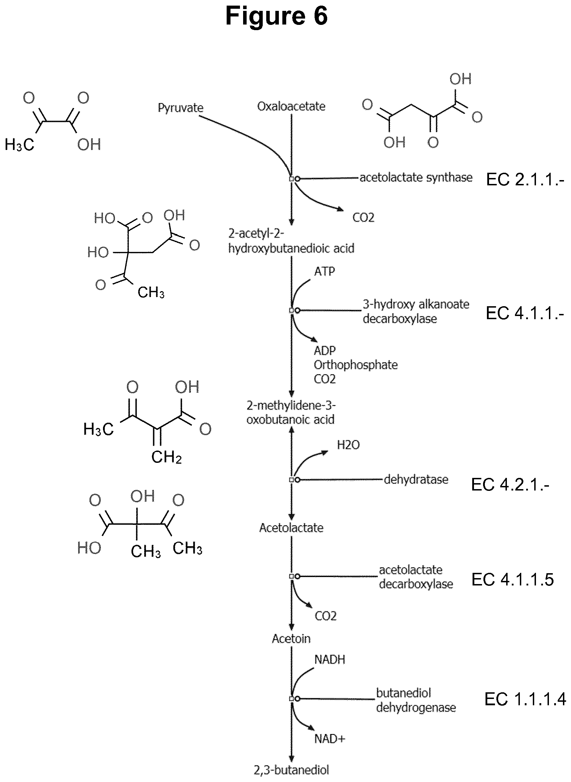

The sixth pathway of 2,3-butanediol biosynthesis (Figure 6) includes steps of condensation of pyruvate and oxaloacetate to 2-acetyl-2-hydroxybutanedioic acid by acetolactate synthase (ALS), conversion of 2-acetyl-2-hydroxybutanedioic acid to 2-methylidene-3-oxobutanoic acid by 3-hydroxy alkanoate decarboxylase, conversion of 2-methylidene-3-oxobutanoic acid to acetolactate by a dehydratase, conversion of acetolactate to acetoin by acetolactate decarboxylase (ALDC) and conversion of acetoin to 2,3-butanediol by butanediol dehydrogenase (BDH).

As an example, the recombinant microorganism disclosed herein expresses genes encoding polypeptides having activity of acetolactate synthase (ALS), 3-hydroxy alkanoate decarboxylase, dehydratase, acetolactate decarboxylase (ALDC), butanediol dehydrogenase (BDH).

Enzymes having acetolactate synthase (ALS) activity for the condensation of pyruvate and oxaloacetate to 2-acetyl-2-hydroxybutanedioic acid are for example enzyme encoded by the *ilvB* gene from *Escherichia coli,* the *ilvH* gene from *Bacillus cereus,* the *ilvH* gene from *Corynebacterium glutamicum,* the *csr1* gene from *Arabidopsis thaliana.*

Enzymes having 3-hydroxy alkanoate decarboxylase activity are for example enzymes encoded by the *mvaD* gene from *Enterococcus faecalis,* the *yeaH* gene from *Lactococcus lactis,* the *NCU02127.1* gene from *Neurospora crassa* or the *MVD1* from *Schizosaccharomyces pombe.*

Enzymes having dehydratase activity for the conversion of 2-methyldene-3-oxobutanoic acid to acetolactate are for example enzymes encoded by the *CSE45_3601* gene from *Citreicella sp. SE45,* the *caiD* gene from *Bordetella parapertussis,* the *abfD* gene from *Porphyromonas gingivalis.*

Enzymes having ALDC activity are for example encoded by genes *budA* from *Klebsiella pneumoniae, budA* from *Klebsiella oxytoca, budA* from *Enterobacter aerogenes, sala* from *Serratia marcescens, alsD* from *Bacillus subtilis subtilis* 168, *alsD* from *Bacillus thuriengiensis* or *PPSC2*_*c2281* from *Bacillus polymyxa.*

Enzymes having BDH activity are encoded by genes: *butA* from *Corynebacterium glutamicum, budC* from *Klebsiella pneumoniae* 342, *budC* from *Klebsiella oxytoca, EAE*_*17490* from *Enterobacter aerogenes, slaC* from *Serratia marcescens, BDH1* and *BDH2* from *Saccharomyces cerevisiae, bdhA* from *Bacillus subtilis subtilis* 168, *BMB171_C0587* from *Bacillus thuriengensis* or *PPSC2_c3335* and *PPSC2_c3890* from *Bacillus polymyxa.*

Any protein encoding a gene having at least 80-85%, preferentially 85-90%, more preferentially 90-95%, even more preferentially 96%, 97%, 98%, 99% sequence identity to any of those genes may be used.

### Optimisations of 2,3-butanediol production

In another aspect of the disclosure, the recombinant microorganism is further modified to improve the availability of pyruvate, by attenuating the degradation pathway of pyruvate in other product than 2,3-butanediol.

In yeast, attenuating lactate production from pyruvate is achieved by deleting, in the recombinant yeast disclosed herein, at least one gene chosen among *DLD1, CYB2, DLD3* and *DLD2.* Preferably the genes *DLD1*, *CYB2, DLD3* and *DLD2* are deleted in the modified yeast.
For attenuating fumarate and succinate production from pyruvate, the *FUM1* gene is attenuated in the modified yeast. The gene *FUM1* seems to be essential for the yeast, thus attenuation is preferred in this example.
For attenuating glycerol production from pyruvate, at least one gene chosen among *HOR2, RHR2, GPD1, GPD2, GUT1* and *TDA10* is deleted in the recombinant yeast disclosed herein. Preferably the genes *HOR2, RHR2, GPD1, GPD2, GUT1* and *TDA10* are deleted in the modified yeast.

In bacterium, optimization of pyruvate availability is achieved by overexpressing, in the bacterium disclosed herein, at least one gene involved in pyruvate biosynthesis pathway, chosen among genes coding for phosphoglycerate mutase (*gpmA* and *pgmI* genes from *Escherichia coli* or homologous gene), enolase (*eno* from *Escherichia coli* or homologous gene) or pyruvate kinase (*pykA* and *pykF* genes from *Escherichia coli* or homologous gene). Alternatively or in combination, at least one gene involved in pyruvate degradation pathway is attenuated. This gene is chosen among pyruvate oxidase (*poxB* from *Escherichia coli* or homologous gene), phosphate acetyltransferase (*pta* from *Escherichia coli* or homologous gene), acetate kinase (*ackA* from *Escherichia coli* or homologous gene) or lactate dehydrogenase (*lldD, ldhA* or *dld* from *Escherichia coli* or homologous gene).

Under fermentative conditions (anaerobia, microaerobia or the presence of large amounts of sugar), yeast that will produce 2.3,-butanediol will only be able to oxidise 50 % of all NADH produced during glycolysis. These electrons need to be transferred to other artificial electron acceptors.

For example, these electron acceptors can be oxidized forms of nitrogen, such as nitrate, nitrite or nitrous oxide, or nitrogen itself. Certain fungi are capable of reducing these nitrogen compounds to ammonia under anaerobic or microaerobic conditions by a process called "ammonia fermentation", as described in Zhou *et al.* (2002) or Takasaki et al. (2004). These low oxygen conditions present an advantage for industrial production since cost of aeration can be eliminated. Thus either the BDO production pathway as described in patent application WO2011041426 will be implemented into an organism capable of performing "ammonia fermentation", or preferentially the fermentation pathway will be transferred into a BDO producing yeast strain. Under certain conditions it may be necessary to express a constitutive ATPase to reduce the amount of ATP in excess in the cell.

In another example, NADH-dependant glyceraldehyde-3-phosphate dehydrogenase is replaced by an NADPH-dependant enzyme. In this case the BDO producing organism will produce an excess of NADPH that in turn can be used via assimilatory nitrate reduction, requiring the introduction of nitrate reductase into certain yeast strains that lack this enzyme.

In another example, other electron accepting substrates can be added. These can be inorganic substrates, such as sulphate and oxidised iron or organic substrate such as for example mannose that will be reduced to mannitol. NADH or NADPH excess can also be used to transform oxidized precursor molecules into reduced products. In this way the excess reducing power can be utilized to generate valuable co-products that can be recovered from the fermentation broth.

It is preferred that the engineered biosynthetic pathway provides at least partial redox balance to the cell. At least partial redox balance may be achieved, for example, by including an enzyme in the engineered biosynthetic pathway that requires NADH for its activity. Utilizing NADH balances production of NADH during conversion of glucose to pyruvate. In wild type cells NADH is utilized in conversion of glucose to glycerol, and in production of ethanol from pyruvate. The present adh- production yeast cells have unbalanced NADH due to disruption of ethanol production. Any method of increasing NADH- dependent enzyme activity in the present production host cell may be used in balancing redox. In addition to including a NADH-dependent enzyme in the biosynthetic pathway, methods include expressing an enzyme that requires NADH but that is not part of the engineered pyruvate-utilizing biosynthetic pathway. A redox-balancing NADH-dependent enzyme may be expressed from a heterologous gene.

Alternatively, expression of an endogenous gene encoding an NADH- dependent enzyme may be increased to provide increased NADH- dependent enzyme activity.

In the disclosure, biosynthesis pathways 1, 5 and 6 are partially redox balanced but pathways 2, 3 and 4 are totally redox balanced.

### Strain optimization to enhance the production of 2,3-butanediol

### Sugar import - Improvement of glucose import

Like bacteria, yeasts consume mono- and disaccharides preferentially to other carbohydrates. In yeasts however, the major sugar transporters work by an energy-independent, facilitated diffusion mechanism. These so-called hexose (Hxt) and galactose (Gal2) transporters naturally exhibit different affinities and specificities for their substrates. They are abundant in *S. cerevisiae* but much less so in other industrial important yeast strains including *Kluveromyces lactis* and *Pichia stipitis* (Boles & Hollenberg, 1997; Wieczorke *et al.,* 1999).

One way to improve the glucose import into the strain producing 2,3-butanediol is to overexpress the genes encoding the hexose and galactose transporters or to modify the genes involved in their complex regulatory pathway, Snf3/Rgt2-Rgt1.

In an example, the recombinant yeast overexpresses at least one gene of the following: *HXT1, HAT2, HXT4, HXT5, HXT7* and *GAL2.*

### Sugar import - ATP consumption

As identified by Larsson and co-workers in 1997, the glycolytic flux is conditionally correlated with ATP concentration in *S. cerevisiae.* ATP has a strong negative effect on glycolytic activity affecting several of the glycolytic enzymes. However, the main targets for ATP inhibition were phosphofructokinase and pyruvate kinase. Other potential candidates as enzymes susceptible to ATP inhibition included hexokinase and enolase (Larsson *et al.,* 1997 and 2000).

For example, the recombinant microorganism producing 2,3-butanediol is further modified to consume its excess of ATP, by overexpressing the soluble part of an ATPase enzyme. This can be accomplished by either overexpressing endogenous genes ATP1, ATP2 and ATP3 of the yeast strain or by introducing heterologous genes, as for instance *atpAGD* from *E.coli.*

In another example, glucose import is improved by expressing at least one desensitized allele of genes known to be inhibited by an excess of ATP such as: *PFK1, PFK2, PYK1, PYK2, HXK1, HXK2, YLR446W, GLK1, ENRO1* and *ENO2.*

### Sugar import - Improvement of C5 sugar import

The import of pentoses by recombinant microorganism is a major issue for industrial process since C5 sugars are major constituents of hydrolysed lignocellulosic biomass. Native strains of *S. cerevisiae,* like many other types of yeast, are unable to utilize either xylose or arabinose as fermentative substrates (Hahn-Hagerdal *et al.,* 2007; Jin *et al.,* 2004). Interestingly, it is able to uptake xylose even though the sugar is not a natural substrate (Hamacher *et al.,* 2002).

*S. cerevisiae* GAL2, HXT1, HXT2, HXT4, HXT5, and HXT7 catalyze the uptake of xylose because they have broad substrate specificity (Hamacher *et al.,* 2002; Saloheimo *et al.,* 2007; Sedlak & Ho 2004). However, their affinity for xylose is much lower than that for glucose and the xylose uptake by the transporters is strongly inhibited by glucose (Saloheimo *et al.,* 2007).

Several changes are needed to obtain a strain able to grow and consume xylose and/or arabinose. These different modifications are a part of the disclosure.

### - Overexpression of heterologous xylose transporters

In order to improve the xylose and arabinose uptake, the recombinant 2,3-BDO producer strain has to be modified to express heterologous genes coding for xylose or arabinose transporters. For example, genes GXF1, SUT1 and AT5g59250 from *Candida intermedia, Pichia stipitis* and *Arabidopsis thaliana,* respectively, are overexpressed to improve xylose utilization by the yeast (Runquist *et al.,* 2010).

### - Overexpression of pathways involved in the metabolism of xylose and arabinose

Yeast strains are able to take up xylose even though the sugar is not a natural substrate. Even though genes for xylose assimilation are present in *S. cerevisiae* they are not expressed at a sufficient level to enable significant sugar assimilation. Thus genetic modifications are necessary to improve the assimilation of pentose sugars. All enzymes that allow the transformation of xylose or arabinose to xylitol need to be enhanced as well as the enzymes which convert xylitol in xylulose, and xylulose into xylulose-5-phosphate.

Either, the homologous genes from the xylose and arabinose pathways have to be overexpressed or heterologous genes from bacteria have to be overexpressed.

For example, the xylose uptake and its assimilation by the strain are improved by overexpressing for example:
1) Genes *XYL1* or *GRE3* coding the aldolase reductase of *P. stipitis* and S. *cerevisiae,* respectively, associated to overexpression of *XYL2* encoding the xylitol dehydrogenase from *P. stipitis,* combined with the overexpression of genes XKS1 or XYL3 encoding the xylulokinase from *S. cerevisiae* and *P. stipitis,* respectively,
2) The gene *xylA* encoding a xylose isomerase from bacteria or *Piromyces* associated to the overexpression of genes *XKS1* or *XYL3* encoding the xylulokinase from *S. cerevisiae* and *P. stipitis,* respectively.

In another example, arabinose uptake and its assimilation by the strain are improved by overexpressing for example:
1) Homologous genes *AYL1* or *GRE3* coding the aldolase reductase of *P. stipitis* and *S. cerevisiae,* respectively, associated to *lad1* encoding the L-arabinitol 4-hydrogenase and *lxr1* encoding a L-xylulose reductase from *Trichoderma reesei,* in combination with the overexpression of *XYL2* encoding the xylitol dehydrogenase from *P.stipitis,* and in addition the overexpression of genes *XKS1* or *XYL3* encoding the xylulokinase from *S. cerevisiae* and *P. stipitis,* respectively,
2) Heterologous genes *araA* and *araB* encoding bacterial arabinose isomerase and ribulose kinase.

### - Optimization of the pentose phosphate pathway

This can be done by overexpressing at least one gene belonging to the non oxidative pentose phosphate pathway; TAL1, TKL1, RKL1 and RPE1 from the yeast strain.

### - Optimization of the availability of NAPDH cofactors required by the enzymes involved in the metabolism of C5-sugars

This is attained by expressing the transhydrogenases of *E. coli* in the yeast strain. The genes *udhA* and or *pntAB* from *E.coli* will be overexpressed in the producer strain.
The carbon source used in this disclosure comprises hexoses or pentoses or a mixture thereof.

### Culture conditions

The invention is also related to a method of production of 2,3-butanediol (BDO) comprising the following steps:
- providing a recombinant microorganism as previously described ,
- cultivating the recombinant microorganism in a culture medium containing a source of carbon, and
- recovering the 2,3-butanediol.

Typically, microorganisms of the invention are grown at a temperature in the range of about 20 °C to about 37 °C in an appropriate medium.

Suitable growth media for yeast are common commercially prepared media such as broth that includes yeast nitrogen base, ammonium sulfate, and dextrose as the carbon/energy source) or YPD Medium, a blend of peptone, yeast extract, and dextrose in optimal proportions for growing most. Other defined or synthetic growth media may also be used and the appropriate medium for growth of the particular microorganism will be known by one skilled in the art of microbiology or fermentation science.

The term an 'appropriate culture medium', as used herein for bacterium, refers to a medium (e.g., a sterile, liquid media) comprising nutrients essential or beneficial to the maintenance and/or growth of the cell such as carbon sources or carbon substrate, nitrogen sources, for example, peptone, yeast extracts, meat extracts, malt extracts, urea, ammonium sulfate, ammonium chloride, ammonium nitrate and ammonium phosphate; phosphorus sources, for example, monopotassium phosphate or dipotassium phosphate; trace elements (e.g., metal salts), for example magnesium salts, cobalt salts and/or manganese salts; as well as growth factors such as amino acids, vitamins, growth promoters, and the like.

As an example of known culture media for *E. coli,* the culture medium can be of identical or similar composition to an M9 medium (Anderson, 1946), an M63 medium (Miller, 1992) or a medium such as defined by Schaefer *et al.* (1999).

Suitable pH ranges for the fermentation are between pH 3.0 to pH 7.5, where pH 4.5 to pH 6.5 is preferred as the initial condition.

Fermentations may be performed under aerobic or anaerobic conditions, where anaerobic or micro-aerobic conditions are preferred.

The amount of product in the fermentation medium can be determined using a number of methods known in the art, for example, high performance liquid chromatography (HPLC) or gas chromatography (GC).

The present process may employ a batch method of fermentation. A classical batch fermentation is a closed system where the composition of the medium is set at the beginning of the fermentation and not subject to artificial alterations during the fermentation. Thus, at the beginning of the fermentation the medium is inoculated with the desired organism or organisms, and fermentation is permitted to occur without adding anything to the system. Typically, however, a "batch" fermentation is batch with respect to the addition of carbon source and attempts are often made at controlling factors such as pH and oxygen concentration. In batch systems the metabolite and biomass compositions of the system change constantly up to the time when the fermentation is stopped. Within batch cultures cells progress through a static lag phase to a high growth log phase and finally to a stationary phase where growth rate is diminished or halted. If untreated, cells in the stationary phase will eventually die. Cells in log phase generally are responsible for the bulk of production of end product or intermediate.

A Fed-Batch system may also be used in the present invention. A Fed-Batch system is similar to a typical batch system with the exception that the carbon source substrate is added in increments as the fermentation progresses. Fed-Batch systems are useful when catabolite repression (e.g. glucose repression) is apt to inhibit the metabolism of the cells and where it is desirable to have limited amounts of substrate in the media. Measurement of the actual substrate concentration in Fed-Batch systems is difficult and is therefore estimated on the basis of the changes of measurable factors such as pH, dissolved oxygen and the partial pressure of waste gases such as CO₂.

Fermentations are common and well known in the art and examples may be found in Sunderland *et al.,* (1992). Although the present invention is performed in batch mode it is contemplated that the method would be adaptable to continuous fermentation.

Continuous fermentation is an open system where a defined fermentation medium is added continuously to a bioreactor and an equal amount of conditioned media is removed simultaneously for processing. Continuous fermentation generally maintains the cultures at a constant high density where cells are primarily in log phase growth.

Continuous fermentation allows for the modulation of one factor or any number of factors that affect cell growth or end product concentration. For example, one method will maintain a limiting nutrient such as the carbon source or nitrogen level at a fixed rate and allow all other parameters to vary. In other systems a number of factors affecting growth can be altered continuously while the cell concentration, measured by media turbidity, is kept constant. Continuous systems strive to maintain steady state growth conditions and thus the cell loss due to the medium being drawn off must be balanced against the cell growth rate in the fermentation. Methods of modulating nutrients and growth factors for continuous fermentation processes as well as techniques for maximizing the rate of product formation are well known in the art of industrial microbiology.

It is contemplated that the present invention may be practiced using either batch, fed-batch or continuous processes and that any known mode of fermentation would be suitable. Additionally, it is contemplated that cells may be immobilized on a substrate as whole cell catalysts and subjected to fermentation conditions for production.

### Purification of 2,3-butanediol

According to a specific aspect of the invention, the fermentative production of 2,3-butanediol comprises a step of isolation of the 2,3-butanediol from the culture medium. Recovering the 2,3-butanediol from the culture medium is a routine task for a man skilled in the art. It may be achieved by a number of techniques well known in the art including but not limiting to distillation, gas-stripping, pervaporation or liquid extraction. The expert in the field knows how to adapt parameters of each technique dependant on the characteristics of the material to be separated.

Distillation may involve an optional component different from the culture medium in order to facilitate the isolation of 2,3-butanediol by forming azeotrope and notably with water. This optional component is an organic solvent such as cyclohexane, pentane, butanol, benzene, toluene, trichloroethylene, octane, diethylether or a mixture thereof.

Gas stripping is achieved with a stripping gas chosen among helium, argon, carbon dioxide, hydrogen, nitrogen or mixture thereof.

Liquid extraction is achieved with organic solvent as the hydrophobic phase such as pentane, hexane, heptane, dodecane.

The purification conditions may be specifically adapted to the downstream transformation of 2,3-BDO to 1,3-butadiene, including keeping several co-products in the partially purified 2,3-BDO.

### EXAMPLES

The present disclosure is further defined in the following examples. It should be understood that these examples, while indicating preferred embodiments of the invention, are given by way of illustration only. From above disclosure and these examples, the man skilled in the art can make various changes of the invention to adapt it to various uses and conditions without modify the essentials means of the invention.

In particular, examples show recombinant *Escherichia coli* (*E. coli*) strains and *Saccharomyces cerevisiae* strains (*S. cerevisiae*), but the genetic modifications described in here can easily be performed on other microorganisms of the same family.

*E. coli* belongs to the *Enterobacteriaceae* family, which comprises members that are Gram-negative, rod-shaped, non-spore forming and are typically 1-5 µm in length. Most members have flagella used to move about, but a few genera are non-motile. Many members of this family are a normal part of the gut flora found in the intestines of humans and other animals, while others are found in water or soil, or are parasites on a variety of different animals and plants. *E. coli* is one of the most important model organisms, but other important members of the *Enterobacteriaceae* family include *Klebsiella,* in particular *Klebsiella terrigena, Klebsiella planticola* or *Klebsiella oxytoca,* and *Salmonella.*

*S. cerevisiae* is a model organism and belongs to the *Saccharomycetaceae* family, which comprises members that are spherical, elliptical or oblong acuminate cells with an asexual reproduction by budding and are typically 5-10 µm in diameter. Many members are used for industrial production due to their fermentative capacity. *S. cerevisiae* is one of the most important model organisms, but other important members of the *Saccharomycetaceae* family include *Pichia, Candida* and *Kluyveromyces,* in particular *Kluyveromyces lactis, Kluyveromyces marxianus* and *Pichia angusta.*

**Table 1: Genotype and corresponding number of intermediates strains and producing strains described in the following examples.**

| **Strain number** | **Génotype** |
|---|---|
| **1 (MG1655)** | F- lambda- *ilvG- rfb*-50 *rph*-1 |
| **2** | MG1655 Δ*poxB* Δ*ldhA* |
| **3** | MG1655 Δ*poxB* Δ*ldhA* Δ*ackA-pta*::Km |
| **4** | MG1655 Δ*poxB* Δ*lphA* Δ*ackA-pta*::Km pCL1920-Ptrc01/E02/RBS01*2-*pdc1sc* pBBRIMCS5-Ptrc01/E02/RBS01*2-*budCkp*-*Ptrc01*/*RBS01* **2-pflB* |
| **5** | MG1655 Δ*poxB* Δ*lphA* Δ*ackA-pta*::Km (pCL1920-Ptrc01/E02/RBS01*2-*pdc1sc*) (pBBR1MCS5-Ptrc01/E02/RBS01*2-*budCkp*-Vtrc01/RBS01*2-*pflB*-Ptrc01/RBS01*2-*mhpF*-TT07) |
| **6** | MG1655 Δ*poxB* Δ*lphA* Ptrc157-*aceEF::*Km |
| **7** | MG1655 Δ*poxB* Δ*ldhA* Ptrc157-*aceEF::*Km (pBBR1MCS5-Ptrc01/E02/RBS01*2-*budCkp-*Ptrc01/RBS01*2-*alsSkpO1ec*-TT07) (pCL1920-Ptrc01/E02/RBS01*2-*ilv6scO1ec*-TT07) |
| **8** | MG1655 Δ*poxB* Δ*lphA* Δ*ackA-pta*::Km Ptrc01/E06/RBS01*2-*pflA::*Cm |
| **9** | MG1655 Δ*poxB* Δ*lphA* Δ*ackA-pta*::Km Ptrc01/E06/RBS01*2-*pflA::*Cm (pCL1920-Ptrc01/E02/RBS01*2-*pdc1sc*) (pBBR1MCS5-Ptrc01/E02/RBS01*2-*budCkp-*Ptrc01/RBS01***2-*pflB*-UTR*-acoABbs*-TT07) |
| **10 (CEN**.**PK2**-**1C)** | *MATa ura3-52 trp1-289 leu2-3,112 his3Δ 1 MAL2-8^{C} SUC2* |
| **11** | *MATa ura3-52 trp1-289 leu2-3,112 his3Δ 1 MAL2*-*8^{C} SUC2 Δadh1 Δadh3 Δadh5*::Gn |
| **12** | *MATa ura3-52 trp1-289 leu2-3,112 his3Δ 1 MAL2*-*8^{C} SUC2 Δadh1 Δadh3* Δ*adh5*::Gn Δ*ilv2*::Hg |
| **13** | *MATa ura3-52 trp1-289 leu2-3,112 his3Δ 1 MAL2*-*8^{C} SUC2* Δ*adh1* Δ*adh3* Δ*adh5*::Gn Δ*ilv2*::Hg (p424-*P*h*xt7-bdh1*-TT*cyc1*) (p426-P*hxt7-mhpFec*-TT*cyc1*) (p423-P*hxt7-pflAec*-TT*cyc1-*P*pgk1-pflBec*-TT*pdc1*) |
| **14** | *MATa ura3-52 trp1-289 leu2-3,112 his3Δ 1 MAL2*-*8^{C} SUC2* Δ*adh1* Δ*adh3* Δ*adh5*::Gn Δ*ilv2*::Hg (p424-P*hxt7-bdh1*-TT*cyc1*) (p426-P*hxt7-acoAbs*-TT*cyc1*-P*pgk1-acoBbs*-TT*pdc1*) (p423-P*hxt7-pflAec*-TT*cyc1*-Ppgk1*-pflBec*-TT*pdc1*) |

### PROTOCOLS:

The *E. coli* strains engineered to produce 2.3BDO were generated using procedures described in patent application WO2010/076324. The gene disruption in specified chromosomal locus was carried out by homologous recombination as described by Datsenko & Wanner (2000). The antibiotic resistant cassette can be amplified on pKD3, pKD4, pKD 13 or any other plasmid containing another antibiotic resistant gene surrounded by FRT sites. Chromosomal modifications were transferred to a given *E. coli* recipient strain by P1 transduction.

The yeast strains engineered to produce 2.3BDO were generated using procedures described by Baudin et al. (1993) and Wach et al. (1994). Chromosomal deletions used PCR-generated deletion strategy to replace yeast open reading frame of interest from its start- to stop- codon with a cassette resistance module (KanMX conferring geneticin resistance or Hph (hygromycin B phosphotransferase) conferring hygromycin B resistance). Plasmid constructions were done by homologous recombination as described by Ma et al. (1987) and Raymond et al (1999).

### Example 1: Construction of strain 5: overexpression of pyruvate decarboxylase, pyruvate formate lyase, acetaldehyde dehydrogenase and butanediol dehydrogenase genes in E. coli

### Construction of strain 2

In order to prevent the accumulation of acetate and the consumption of pyruvate during *E. coli* cells culture, *poxB* gene encoding pyruvate oxidase was deleted by using primers DpoxB-F (SEQ ID N°01) and DpoxB-R (SEQ ID N°02).

In order to prevent the accumulation of lactate and the consumption of pyruvate during *E. coli* cells culture, *ldhA* gene encoding lactate dehydrogenase was deleted by using primers DldhA-F (SEQ ID N°03) and DldhA-R (SEQ ID N°04).

Both deletions were introduced in MG1655 using homologous recombination method in *Escherichia coli* (Datsenko & Wanner (2000)). This method allowed the combination of multiple genetic modifications using different antibiotic resistance genes which can be removed. The resulting strain MG1655 *ΔpoxB Δlpha* was called strain 2 (Table 1).

### Construction of strain 3

In order to prevent the accumulation of acetate during the culture, the deletion of *ackA-pta* transcription unit was performed. The acetate production by AckA-Pta pathway contains two enzymes: the phosphotransacetylase (*pta*) that reversibly converts acetyl-CoA, and inorganic phosphate to acetyl phosphate and CoA, and the acetate kinase (*ackA*) that reversibly converts acetyl phosphate and ADP to acetate and ATP.
The *ackA-pta* transcription unit was deleted by using primers DackA-pta-F (SEQ ID N°05) and DackA-pta-R (SEQ ID N°06) and introduced into strain 2 by PI transduction. The validated strain MG1655 *ΔpoxB Δlpha ΔackA-pta::Km* was called strain 3 (Table1)

### Construction of strain 4

### Construction of plasmid pCL1920-Ptrc01/E02/RBS01*2-pdc1sc

The plasmid pCL1920-Ptrc01/E02/RBS01*2-*PDC1sc* was derived from pCL1920 (Lerner & Inouye, 1990, NAR 18, 15 p 4631), *PDC1sc* gene encoding the pyruvate decarboxylase from *Saccharomyces cerevisiae* S288C which expression was driven by a constitutive P*trc* promoter and an optimized RBS sequence. The *PDC1sc* gene was PCR-amplified with primers Ptrc01/E02/RBS01*2-pdc1sc F (SEQ ID N°07) and pdc1sc R (SEQ ID N°08) using *Saccharomyces cerevisiae* reference strain S288C genomic DNA and cloned between the *SacI* and *BamHI* restriction sites of pCL1920 plasmid. The resulting plasmid was called *pCL1920-Ptrc01*/*E02*/*RBS01*2-pdc1sc.*

### Construction of plasmid pBBR1MCS5-Ptrc01/E02/RBS01*2-budCkp-Ptrc01/RBS01*2-pflB

The pBBR1MCS5-*Ptrc01*/*E02*/*RBS01*2-budCkp-Ptrc01*/*RBS01*2-pflB* plasmid is derived from pBBR1MCS5 plasmid (M. E. Kovach, (1995), Gene 166:175-176), *budCkp* synthetic gene encoding butanediol dehydrogenase and *pflB* gene *from E. coli* encoding the pyruvate formate lyase. In this plasmid, expression of the *budCkp* synthetic gene and *pflB* gene were driven by independents constitutive *trc* promoters and optimized RBS sequences.

### Synthetic gene budC

A *budC* synthetic gene from *Klebsiella pneumoniae MGH78578* coding for the butanediol dehydrogenase (acetoin reductase) was synthesized by Life Technologies (SEQ ID N°54). The construct was cloned into the supplier's pM vector and verified by sequencing.

### Construction of pBBR1MCS5-Ptrc01/E02/RBS01*2-budCkp plasmid

The Ptrc01/E02/RBS01*2-*budCkp* fragment was PCR-amplified with primers Ptrc01/E02/RBS01*2-budCkp F (SEQ ID N°09) and budCkp R (SEQ ID N°10) using the pMA-T-*budCkp* vector provided by the supplier and cloned between the *SacI* and *Xba*I sites of the pBBRIMCS5 plasmid. The resulting plasmid was called pBBR1MCS5-*Ptrc01IE02*/*RBS01 *2-budCkp.*

### Construction of the plasmid pBBR1MCS5-Ptrc01/E02/RBS01*2-budCkp-Ptrc01/RBS01 *2-pflB

In order to increase the acetyl-CoA pool in the cell, *pflB* gene from *E. coli* was over-expressed. This enzyme catalyses by an oxygen-sensitive radical-based reaction the acetyl-CoA synthesis from pyruvate without ATP consumption.

*"Ptrc01*/*RBS01*2-pflB"* fragment was PCR-amplified with primers Ptrc01-pflB-SpeI F (SEQ ID N°11) and pflB-EcoRI R (SEQ ID N°12) using *E. coli* MG1655 genomic DNA and cloned between the *SpeI* and *EcoR*I sites of the *pBBR1MCS5-Ptrc01*/*E02*/*RBS01***2-budCkp* plasmid described above. The resulting plasmid was called pBBR1MCS5-P*trc01*/*E02*/*RBS01*2-budCkp-*P*trc01*/*RBS01*2-pflB.*

### Construction of strain 4

The *pCL1920-Ptrc01*/*E02*/*RBS01*2-pdc1sc* and *pBBR1MCS5-Ptrc01*/*E02*/*RBS01*2-budCkp-Ptrc01*/*RBS01*2-pflB* were introduced by electroporation into strain 3 (Table 1). The resulting strain MG1655 *ΔpoxB Δlpha ΔackA-pta::Km* (pCL1920-*Ptrc01*/*E02*/*RBS01*2-pdc1sc)* (pBBR1MCS5-Ptrc01/E02/RBS01*2-*budCkp-*Ptrc01/RBS0 *2-*pflB*) was called strain 4 (Table 1).

### Construction of strain 5

### Construction of plasmid pBBR1MCS5-Ptrc01/E02/RBS01*2-budCkp-Ptrc01/RBS01*2-pf1B-Ptrc01/RBS01*2-mhpF-TT07

The plasmid pBBR1MCS5-Ptrc01/E02/RBS0 *2-*budCkp*-Ptrc01/RBS01 *2-*pflB-Ptrc01*/*RBS01*2-mhpF-TT07* was derived from pBBR1MCS5-Ptrc01/E02/RBS01*2-*budCkp-Ptvc01*/*RBS01*2-pflB* described above and *mhpF* gene encoding an acetaldehyde dehydrogenase from *E. coli.*
In this plasmid, expression of *mhpF* gene was driven by a constitutive *trc* promoter and optimized RBS sequence. A transcriptional terminator was added at the end of the construct, downstream *mhpF* gene.
*mhpF* gene was PCR-amplified with primers Ptrc01-mhpF F (SEQ ID N°13) and Ptrc01-mhpF-TT07 R (SEQ ID N°14) using *E. coli* MG1655 genomic DNA and cloned into the *ApaI* and *EcoR*I restriction sites of the *pBBR1MCS5-Ptrc01*/*E02*/*RBS01*2-budCkp-Ptrc01*/*RBS01*2-pflB* plasmid (described above). The resulting plasmid was called *pBBR1MCS5-Ptrc01*/*E02*/*RBS01*2-budCkp-Ptrc01*/*RBS01 *2-pflB-Ptrc01*/*RBS01 *2-mhpF*-TT07.

### Construction of strain 5

The engineered strain which exhibits an increased 2,3-BDO production expressed the *pflB* gene to produce acetyl-CoA, the *mhpF* gene to convert acetyl-CoA into acetaldehyde, the *PDC1* from *Saccharomyces cerevisiae* to convert pyruvate and acetaldehyde to acetoin and the synthetic *budCkp* gene to finally convert acetoin into 2,3-butanediol.

The pCL1920-Ptrc01/E02/RBS01*2-*pdclsc* and pBBR1MCS5-Ptrc01/E02/RBS01*2-*budCkp*-Ptrc01/RBS01*2-*pflB-*Ptrc01/RBS01*2*-mhpF*-TT07 were introduced by electroporation into strain 3 (Table1). The resulting strain MG1655 Δ*poxB* Δ*lpha* Δ*ackA-pta*::Km (pCL1920-P*trc01*/*E02*/*RBS01**2-*pdclsc)* (pBBR1MCS5-P*trc01*/*E02*/*RBS01*2-budCkp-*P*trc01*/*RBS01*2-pflB-Ptrc01*/*RBS01 *2-mhpF-TT07)* was called strain 5 (Table 1).

### Reference Example 2: Construction of strain 7: overexpression of acetolactate synthase, diacetyl synthase and butanediol dehydrogenase genes in E. coli

### Construction of strain 6

In order to keep the pool of pyruvate which is a precursor of the 2,3BDO, the expression of *aceEF* operon encoding pyruvate dehydrogenase subunit E1 and E2 respectively was attenuated by adding a weak *trc* constitutive promoter and a transcriptional terminator upstream the *aceE* gene and by using Ptrc157-aceE F (SEQ ID N°15) and Ptrc157-aceE R (SEQ ID N°16).
Chromosomal attenuation of *aceEF* operon was introduced into MG1655 using homologous recombination (Datsenko & Wanner (2000)) and then transferred by PI transduction in strain 2 (Table1). The resulting strain MG1655 Δ*poxB* Δ*lpha* Ptrc157-*aceEF*::Km was called strain 6 (Table 1).

### Construction of strain 7

### Construction of plasmid pBBR1MCS5-Ptrc01/E02/RBS01*2-budCkp-Ptrc01/RBS01*2-alsSkpO1ec-TT07

Plasmid *pBBR1MCS5-Ptrc01*/*E02*/*RBS01*2-budCkp-*P*trc01*/*RBS01*2-alsSkpOlec-*TT07 was derived from pBBR 1MCS5-P*trc01*/*E02*/*RBS01*2-budCkp* described in Example 1 and *alsSkpO1ec* synthetic gene which expression was driven by an independent constitutive *trc* promoter and an optimized RBS sequence. A transcriptional terminator was added at the end of the construct, downstream *alsSkpO1ec* gene.

### Synthetic gene alsSkpO1ec

A synthetic gene of the *alsS* gene from *Klebsiella pneumoniae 342* coding for the acetolactate synthase (KPK_2270) was synthesized by Life Technologies (SEQ ID N°55). The construct was cloned into the supplier's pM vector and verified by sequencing:
The *alsSkpO1ec* synthetic gene was PCR-amplified with primers Ptrc01/RBS01*2-alsSkpOlec F (SEQ ID N°17) and EcoRI-alsSkpO1ec-TT07 R (SEQ ID N°18) by using the *pMK-alsSkpO1ec* plasmid provided by the supplier and cloned between the *BamHI* and *EcoR*I sites of the pBBR1MCS5-Ptrc01/E02/RBS01*2-*budCkp* plasmid. The resulting plasmid was called *pBBR1MCS5-Ptrc01*/*E02*/*RBS01*2-budCkp- Ptrc01*/*RBS01*2-alsSkpO1ec-TT07.*

### Construction of plasmid pCL1920-Ptrc01/E02/RBS01*2-ilv6scO1ec-TT07

Plasmid pCL1920-P*trc01*/*E02*/*RBS01*2-ilv6scO1ec*-TT07 was derived from pCL1920 (Lerner & Inouye, 1990, NAR 18, 15 p 4631) and *ilv6cO1ec* synthetic gene encoding diacetyl synthase from *Saccharomyces cerevisiae S288C,* which expression was driven by a constitutive *trc* promoter and optimized RBS sequence. A transcriptional terminator was added at the end of the construct.

### Synthetic gene ilv6cO1ec

Synthetic gene of the *ILV6* gene from *Saccharomyces cerevisiae* S288C coding for the diacetyl synthase small subunit (P25605) was synthesized by Life Technologies (SEQ ID N°56). The construct was cloned into the supplier's pM vector and verified by sequencing. The *ilv6cO1ec* synthetic gene was PCR-amplified with primers Ptrc01/E02/RBS01*2-ilv6cO1ec F (SEQ ID N°19) and ilv6scO1ec-TT07 R (SEQ ID N°20) using the pMA-*ilv6cO1ec* plasmid provided by the supplier and cloned between the *SacI* and *Kpn*I sites of the pCL1920 plasmid. The resulting plasmid was called pCL1920-Ptrc01/E02/RBS01*2-*ilv6scO1ec-*TT07.

### Construction of strain 7

The engineered strain which exhibits an increased 2,3-BDO production expressed the optimized *alsS* gene from *Klebsiella pneumoniae* to produce acetolactate from pyruvate, the optimized *ILV6* gene from *Saccharomyces cerevisiae* to convert acetolactate into diactetyl and the *budC* gene from *Klebsiella pneumoniae* to finally convert diacetyl into acetoin and then into 2,3-butanediol.

Both plasmids pBBR1MCS5-Ptrc01/E02/RBS01*2*-budCkp*-Ptrc01/RBS01*2-*alsSkpO1ec*-TT07 and pCL1920-Ptrc01/E02/RBS01*2-*ilv6scO1ec-*TT07 were introduced by electroporation into strain 6 (Table 1). The resulting strain MG1655 Δ*poxB* Δ*lpha* P*trc*157*-aceEF*::Km (pBBR1MCS5-Ptrc01/E02/RBS01*2-*budCkp*-Ptrc01/RBS01*2-*alsSkpO1ec-*TT07*)* (pCL1920-Ptrc01/E02/RBS01*2*-ilv6scO1ec*-TT07) was called strain 7 (Table 1).

### Reference Example 3: Construction of strain 9: overexpression of pyruvate decarboxylase, pyruvate formate lyase, acetoin dehydrogenase and butanediol dehydrogenase genes in E. coli

### Construction of strain 8

In order to increase acetyl-CoA production from pyruvate, pyruvate formate lyase (PFL) *pflB* gene is over-expressed. Since PFL is working as a dimer of PflB, whose maturation requires the enzyme PflAE, the *pflA* gene was over-expressed. For this purpose, a strong constitutive promoter *trc* and a RBS consensus sequence was inserted in front of *pflA* open reading frame at the chromosome by using homologous recombination method described by Datsenko & Wanner (2000).

Overlapping PCR between resistance cassette Cm fragment (Ptrc01/E06/RBS01*2-pflA F (SEQ ID N°21) and Ptrc01-pflA R (SEQ ID N°22) and fragment homologous to the beginning of *pflA* gene (pflA R (SEQ ID N°23) and pflA F (SEQ ID N°24) was done by using pflA F (SEQ ID N°24) and RI pflB R (SEQ ID N°25) was performed and introduced in MG1655 strain. Chromosomal modification of *pflA* promoter was then transferred by P1 transduction in strain 3 (Table1). The resulting strain MG1655 Δ*poxB* Δ*lpha* Δ*ackA-pta*::Km Ptrc01/E06/RBS01*2-*pfl*A::Cm was called strain 8 (Table1)

### Construction of strain 9

### Construction of plasmid pBBR1MCS5-Ptrc01/E02/RBS01*2-budCkp-Ptrc01/RBS01*2-pflB-UTR-acoABbs-TT07

The plasmid pBBR1MCS5-Ptrc01/E02/RBS0 1*2-*budCkp*-Ptrc01/RBS01 *2-*pflB*-UTR-*acoABbs*-TT07 was derived from the pBBR1MCS5-Ptrc01/E02/RBS01*2-*budCkp-*Ptrc01/RBS01*2-*pflB* plasmid described in Example 1 and *acoAlacoB* operon from *Bacillus subtilis* 168 encoding the acetoin dehydrogenase subunits, alpha and beta respectively which expression was driven by the same constitutive P*trc* promoter than *pflB* gene and their own 5' untranslated region. A transcriptional terminator was added at the end of the construct.

### Construction of pBBR1MCS5-Ptrc01/E02/RBS01*2-budCkp-Ptrc01/RBS01*2-pflB-UTR-acoABbs-TT 07 plasmid

The *UTR-acoABbs*-TT07 fragment was PCR-amplified with primers Ptrc01/UTR-acoAB IF F (2) (SEQ ID N°26) and acoABbs-TT07 IF R (SEQ ID N°27) using *Bacillus subtilis 168* genomic DNA and cloned into the *Hind*III and *Eco*RI restriction sites of the pBBR1MCS5-Ptrc01/E02/RBS01*2-*budCkp-*Ptrc01/RBS01*2-*pflB* plasmid (described in Example 1). The resulting plasmid was called pBBR1MCS5-Ptrc01/E02/RBS01*2-*budCkp-*Ptrc01/RBS01*2-*pflB-UTR-acoABbs*-TT07.

### Construction of strain 9

The engineered strain which exhibits an increased 2,3-BDO production expressed the *pflB* and *pflA* genes to produce acetyl-CoA, the *PDC1* from *Saccharomyces cerevisiae* to convert pyruvate into acetaldehyde, the natural *acoA* and *acoB* genes from *Bacillus subtilis* to condense acetyl-CoA and acetaldehyde into acetoin and the optimized *budC* gene from *Klebsiella pneumoniae* to finally convert acetoin into 2,3-butanediol.

Plasmids pCL1920-Ptrc01/E02/RBS01*2-*pdc1sc* and pBBR1MCS5-Ptrc01/E02/RBS01*2-*budCkp*-Ptrc01/RBS01*2-*pflB*-UTR-*acoABbs*-TT07 were both introduced by electroporation into strain 8 (Table 1). The resulting strain MG1655 Δ*poxB* Δ*ldhA* Δ*ackA-pta::Km* Ptrc01/E06/RBS01*2-*pflA::*Cm (pCL1920-Ptrc01/E02/RBS01*2-*pdc1sc)* (pBBR1MCS5-Ptrc01/E02/RBS01*2-*budCkp*-Ptrc01/RBS01*2*-pflB*-UTR-*acoABbs*-TT07) was called strain 9 (Table 1).

### Example 4: Construction of strain 13: overexpression of pyruvate formate lyase, pyruvate formate lyase activating enzyme, acetaldehyde dehydrogenase and butanediol dehydrogenase genes in S. cerevisiae

### Construction of strain 11

In order to prevent ethanol production, deletions of genes *ADH1, ADH3* and *ADH5* encoding the three major alcohol dehydrogenases were constructed successively by employing the lox::kanMX::loxP/Cre recombinase system and the short flanking homology PCR technology as described by Guldener *et al.* (1996).

Each gene was deleted by introducing a gene deletion cassette containing loxP-kanMX-loxP conferring geneticin resistance and homologous region upstream and downstream the coding region of the gene of interest. Each deletion cassette was PCR-amplified from plasmid pUG6 (Guldener *et al.,* 1996) with primers adh1::kanMXlox fwd (SEQ ID N°28) and adh1::kanMXloxrev (SEQ ID N°29) for *ADH1* gene deletion, adh3::kanMXlox fwd (SEQ ID N°30) and adh3::kanMXloxrev (SEQ ID N°31) for *ADH3* gene deletion and Adh5::kanMXlox fwd (SEQ ID N°32) and Adh5::KanMXloxrev (SEQ ID N°33) for *ADH5* gene deletion. PCR products were transformed into yeast as described by Gietz and al. (2002 and 2007). The loxP-*kanMX*-loxP selection cassette at ADH1 locus and then at ADH3 locus was removed *via* transformation with pSH47 encoding galactose-inducible Cre recombinase (Guldener *et al.,* 1996). The selected and verified strain CEN.PK2-1C Δ*adhl* Δ*adh3* Δ*adh5*::Gn was called strain 11.(Table 1).

### Construction of strain 12

In order to eliminate the 2.3BDO natural production pathway in yeast, *ILV2* gene deletion which encodes the catalytic subunit of the acetolactate synthase has been carried out as described previously, except that, instead of geneticin resistance cassette, hygromycine B resistance (*hphNT1)* resistance cassette was amplified by using ilv2::hphNT1loxrfwd (SEQ ID N°34) and ilv2::hphNT1loxrev (SEQ ID N°35) primers. PCR product was transformed into strain 11 (Table 1) as described by Gietz *et al.* (2002 and 2007). The selected and verified strain CEN.PK2-1C Δ*adhl* Δ*adh3* Δ*adh5*::Gn Δ*ilv2*::Hg was called strain 12 (Table 1).

### Construction of strain 13

### Construction of plasmid p424-Phxt7-bdh1-TTcyc1

The plasmid p424-P*hxt7-bdh1*-TT*cyc1* was derived from p424H7 plasmid (Wieczorke *et al.* 1999 and Hamacher *et al.* 2002) and *BDH1* gene encoding the most active 2,3-butanediol dehydrogenase under the control of truncated *HXT7* promoter and *CYC1* terminator was added at the end of the gene for transcription termination.
Coding region of BDH1 was PCR-amplified with A-BDH1-f (SEQ ID N°36) and A-BDH1-r (SEQ ID N°37) from *S. cerevisiae* S288C genomic DNA and cloned by recombination cloning into the p424H7 plasmid digested by *Bam*HI/*Xho*I. The verified and sequenced plasmid was called p424-Phxt7-*bdh1*-TTcyc1.

### Construction of plasmid p426-Phxt7-mhpFec-TTcyc1

The plasmid p426-P*hxt7-mhpFec-*TT*cyc1* was derived from p426H7 plasmid (Wieczorke *et al.* 1999 and Hamacher *et al.* 2002) and *mhpF* gene encoding the acetaldehyde dehydrogenase from *Escherichia coli* under the control of truncated *HXT7* promoter and the *CYC1* terminator was added at the end of the gene for transcription termination.
Coding region of *mhpF* gene was PCR-amplified with mhpF-f (SEQ ID N°38) and mhpF-r (SEQ ID N°39) from *E. coli MG1655* genomic DNA and cloned by recombination cloning into the p426H7 plasmid digested by *Bam*HI/*Xho*I*.* The verified and sequenced plasmid was called p426-P*hxt7-mhpFec-*TT*cyc1.*

### Construction of plasmid p423-Phxt7-pflAec-TTcyc1-Ppgk41-pflBec-TTpdc1

The plasmid p423-P*hxt7-pflAec*-TT*cyc1-*P*pgk1-pflBec-*TT*pdc1* was derived from p423H7 plasmid (Wieczorke *et al.* 1999 and Hamacher *et al.* 2002). The gene *pflA* gene encoding the pyruvate formate lyase activating enzyme from *Escherichia coli* was cloned under the control of truncated *HXT7* promoter and the *pflB* gene encoding the pyruvate formate lyase cloned under the control of *PGK1* promoter. The *CYC1* and *PDC1* terminators were respectively added at the end of the *pflA* and *pflB* genes for transcription termination.

Coding regions *of pflA and pflB* genes were PCR-amplified with the following primers:
- pflA-f (SEQ ID N°40) and pflA-r (SEQ ID N°41);
- A-EcPFLB-f (SEQ ID N°42) and A-EcPFLB-r (SEQ ID N°43), respectively by using *E. coli* MG1655 genomic DNA.

*CYC1* and *PDC1* terminators and *PGK1* promoter were amplified with the following primers:
- A-tCYC1-f(SEQ ID N°44) and A-tCYC1-r (SEQ ID N°45)
- A-tPDC1-f(SEQ ID N°46) and A-tPDC1-r (SEQ ID N°47)
- A-pPGK1-f (SEQ ID N°48) and A-pPGK1-r (SEQ ID N°49), respectively by using *S. cerevisiae* S288C genomic DNA.

Assembly of each PCR fragment into the p423H7 plasmid digested by *Bam*HI/*Psi*I was done by recombination cloning. The verified and sequenced plasmid was called p423-P*hxt7-pflAec-*TT*cyc1*-P*pgk1-pflBec-*TT*pdc1.*

### Construction of strain 13

The engineered strain which exhibits an increased 2,3-BDO production expressed the *pflB* and *pflA* genes from *E. coli* to produce acetyl-CoA, the *mhpF* gene from *E. coli* to convert acetyl-CoA into acetaldehyde, the endogenous expression of *PDC1* gene to convert pyruvate and acetaldehyde into acetoin and the overexpressed *BDH1* gene to finally convert acetoin into 2,3-butanediol.

Plasmids p424-P*hxt7-bdh1-*TT*cyc1,* p426-P*hxt7-mhpFec-*TT*cyc1* and p423-P*hxt7-pflAec-*TT*cyc1-*P*pgk1-pflBec-*TT*pdc1* were introduced into strain 12 (Table 1) by transformation according to Gietz *et al.,* (2007) protocol. The resulting strain CEN.PK2-1C *Δadh1 Δadh3 Δadh5*::Gn *Dilv2*::Hg (p424-P*hxt7-bdh1-*TT*cyc1*) *(p426-Phxt7-mhpFec-*TT*cyc1*) (p423-P*hxt7-pflAec-*TT*cyc1-*P*pgk1-pflBec-*TT*pdc1*) was called strain 13

### Example 5: Construction of strain 14: overexpression of pyruvate formate lyase, pyruvate formate lyase activating enzyme, acetoin reductase and butanediol dehydrogenase genes in S. cerevisiae

### Construction of strain 14

### Construction of plasmid p426-Phxt7-acoAbs-TTcyc1-Ppgk1-acoBbs-TTpdc1

In order to improve the condensation of acetyl-CoA and acetaldehyde into acetoin, the acetoin reduction complex *acoAB* from *Bacillus subtilis* was overexpressed.

The plasmid p426-P*hxt7*-*acoAbs*-TT*cyc1*-P*pgk1*-*acoBbs*-TT*pdc1* was derived from p426H7 plasmid (Wieczorke *et al.* 1999 and Hamacher *et al.* 2002) and *acoAbs* / *acoBbs* genes under the control of truncated *HXT7* and *PGK1* promoters respectively. The *CYC1* and *PDC1* terminators were added, respectively, at the end of the *acoA* and *acoB* gene for transcription termination.
Coding region of *acoA and acoB* genes were PCR-amplified with the following primers:
- A-acoA-f (SEQ ID N°50) and A-acoA-r (SEQ ID N°51)
- A-acoB-f (SEQ ID N°52) and acoB-r (SEQ ID N°53) respectively by using *Bacillus subtilis 168* genomic DNA.
*CYC1* and *PDC1* terminators and *PGK1* promoter were amplified as described in example 4.

Assembly of each PCR fragment into the p426H7 plasmid digested by *Bam*HI/*Kpn*I was done by recombination cloning. The verified and sequenced plasmid was called p426-P*hxt7-acoAbs*-TT*cyc1-*P*pgk1-acoBbs*-TT*pdc1.*

### Construction of strain 14

The engineered strain which exhibits an increased 2,3-BDO production expressed the *pflB and pflA* genes to produce acetyl-CoA, the endogenous expression of *PDC1* to convert pyruvate to acetoin, the *acoA* and *acoB* genes from *Bacillus subtilis* to condense acetyl-CoA and acetaldehyde into acetoin and the overexpressed *BDH1* gene to finally convert acetoin into 2,3-butanediol.
Plasmids p*424-*P*hxt7-bdh1-*TT*cyc1,* p426-P*hxt7*-*acoAbs*-TT*cyc1*-P*pgk1*-*acoBbs*-TT*pdc1* and p423-P*hxt7-pflAec-*TT*cyc1-*P*pgk1-pflBec-*TT*pdc1* were introduced into strain 12 (Table 1) by transformation according to Gietz *et al.* (2007) protocol. The resulting strain CEN.PK2-1C Δ*adh1* Δ*ads3* Δ*adh5*::Gn *Dilv2*::Hg (p424-P*hxt7-bdh1-*TT*cyc1*) (p426-P*hxt7-acoAbs-*TT*cyc1-*P*pgk1-acoBbs-*TT*pdc1)* (p423-P*hxt7-pflAec-*TT*cyc1-*P*pgk1-pflBec-*TT*pdc1*) was called strain 14 (Table 1).

### Example 6: Production of 2.3BDO in shake flask

### E. coli

### Pathway 3

Production strains were evaluated in small Erlenmeyer flasks using modified M9 medium (Anderson, 1946) that is supplemented with 5 g.L⁻¹ of yeast extract, 10 g.L⁻¹ MOPS and 10 g.L⁻¹ glucose and adjusted to pH 6.8.

A 25 mL preculture was grown at 37°C for 15 hours in a mixed medium (10 % LB medium (Sigma 25 %) with 2.5 g.L⁻¹ glucose and 90 % minimal medium described above). It was used to inoculate a 50 mL culture to an OD₆₀₀ of 0.3 in modified minimal medium.

When necessary, antibiotics were added at concentrations of 50 mg.L⁻¹ for kanamycin and spectinomycin and 10 mg.L⁻¹ for gentamycin. The temperature of the cultures is 37°C and the agitation is set up at 200 RPM. When the culture reached an OD₆₀₀ of 5 to 6, extracellular metabolites were analyzed using HPLC with refractometric detection (organic acids and glucose). Production of BDO was determined by LCMSMS.

**Table 2: BDO titer for strains 1 and 7 with aerobic conditions. For each strain, two repetitions were made.**

| **Strain** | **[BDO] (mg.L⁻¹)** |
|---|---|
| 1 | Not detected |
| 7 | 41 |

As can be seen in table 2, BDO production is increased upon overexpression of ILV6, *budC* and *alsS.*

### Pathways 2 and 4

Production strains were evaluated in small flasks using modified MAC medium that is supplemented with 10 g.L⁻¹ glucose and adjusted to pH 6.8.
A 25 mL preculture was grown at 37°C for 15 hours in a mixed medium (10 % LB medium (Sigma 25 %) with 2.5 g.L⁻¹ glucose and 90 % M9 medium (Anderson, 1946). It was used to inoculate a 50 mL culture to an OD₆₀₀ of 0.3 in MAC medium. The composition of this medium was in g per liter: glycerol: 20.0 ; tryptone: 10.0 ; NaCl*: 5.0 ; NaNO₃ 0.085 ; yeast extract: 5.0 ; K₂HPO₄: 0.5 ; FeSO₄.7H₂O: 0.05 ; HEPES: 23.0 and NTA: 0.2.
When necessary, antibiotics were added at concentrations of 50 mg.L⁻¹ for kanamycin and spectinomycin, of 30 mg.L⁻¹ for chloramphenicol and 10 mg.L⁻¹ for gentamycin. The temperature of the cultures is 37°C, and the agitation set up 200 RPM. Microaerobic conditions were applied to the culture by sealing flasks with rubber stoppers. After 7 hours, extracellular metabolites were analyzed using HPLC with refractometric detection (organic acids and glucose). Production of BDO was determined by LCMSMS. Each strain was evaluated twice.

**Table 3 : BDO titer for strains 1, 4 and 9 with microaerobic conditions for pathway 4**

| **Strain** | **[BDO] (mg.L⁻¹)** |
|---|---|
| 1 | 0 |
| 9 | 118 |
| 4 | 111 |

**Table 4: BDO titer for strains 1, 4 and 5 with microaerobic conditions for pathway 2. For each strain, two repetitions were made.**

| **Strain** | **[BDO] (mg.L⁻¹)** |
|---|---|
| 1 | 0 |
| 5 | **257** |
| 4 | **211** |

BDO production is increased with overexpression of acetolactate synthase, diacetyl synthase and butanediol dehydrogenase genes in *E. coli.*

### S. cerevisiae yeast

Production strains were assessed in small flasks. A 7.5 mL preculture was grown at 30°C for 17 hours in YPD medium (10 g.L⁻¹ yeast extract, 10 g.L⁻¹ Bactopeptone and 10 g.L⁻¹ glucose). It was used to inoculate a 70 mL culture of MM1 medium to reach an OD₆₀₀ of 0.2. Synthetic mineral medium (MM1) was prepared as follows (all concentrations in g.L⁻¹): glucose: 10.0 ; (NH₄)₂SO₄: 11.0 ; KH₂PO₄: 5.5 ; MgSO₄.7H₂O: 0.9 ; EDTA.2Na.2H₂O: 0.0300 ; ZnSO₄.7H₂O: 0.0090 ; CoCl₂ 6H₂O: 0.0006 ; MnCl₂.4H₂O: 0.0020; CuSO₄.5H₂O: 0.0006 ; FeSO₄.7H₂O: 0.0060 ; Na₂MoSO₄.2H2O: 0.0080 ; H₃BO₃: 0.0020 ; CaCl₂.2H₂O: 0.0090 ; biotin: 0.0020 ; calcium pantothenate: 0.0020 ; nicotinic acid: 0.0020 ; myoinositol: 0.0050 ; hydrochloride thiamine: 0.0020 ; para-aminobenzoic acid: 0.0004 and pyridoxine: 0.0020.

When necessary, leucine is added at a final concentration of 0.44 mM in the medium.

The temperature of the culture was maintained at 30°C and with an agitation of 200 RPM. Yeasts were cultivated in aerobic, microaerobic and anaerobic conditions. Microaerobic conditions were applied to the culture by sealing flasks with rubber stoppers. For anaerobic conditions, 70 mL of minimal media were supplemented with 0.42 g.L⁻¹ Tween 80 and 0.04 g.L⁻¹ cholesterol. Bottles were purge with pure nitrogen gas for 15 minutes to establish anaerobic conditions. After about 45 hours, BDO was quantified by LCMSMS and other relevant metabolites were analyzed using HPLC with refractometric detection (organic acids and glucose).
With strains 13 and 14, BDO production was effective for all the evaluated conditions. Final concentrations were comprised between 80 and 130 mg.L⁻¹ for anaerobic conditions and between 187 and 234 mg.L⁻¹ for aerobic conditions. Performances obtained in microaerobic conditions are presented in table 5.

**Table 5: BDO and ethanol titers for strains 10, 13 and 14 in microaerobic conditions. For each strain, two repetitions were made.**

| **Strain** | **BDO (mg.L⁻¹)** | **Ethanol (g.L⁻¹)** |
|---|---|---|
| 10 | 6 | **3.9** |
| 13 | **307** | 0.3 |
| 14 | **341** | 0.5 |

As can be seen in table 5, BDO production is increased upon overexpression of the two pathways introduced in *S. cerevisiae.*

### REFERENCES

### Patent references

WO2011041426
WO2010151525
WO2004076659

### Non-patent references

- Altschul SF (1993), J Mol Evol. 36(3):290-300.
- Anderson EH (1946), Proc. Natl. Acad. Sci. USA 32:120-128.
- Bassit N, Boquien CY, Picque D, Corrieu G. (1993), Appl Environ Microbiol. 59(6):1893-7.
- Baudin A, Ozier-Kalogeropoulos O, Denouel A, Lacroute F, Cullin C. (1993), Nucleic Acids Res. 21(14):3329-3330
- Brat D, Weber C, Lorenzen W, Bode HB, Boles E. (2012), Biotechnology for Biofuels. 5:65-80.
- Boles E, Hollenberg CP. (1997) FEMS Microbiol Rev. 21(1):85-111.
- Celinska E, Grajek W. (2009), Biotechnol Adv. 27(6):715-25.
- Danner H, Braun R. (1999), Chem. Soc. Rev., 28:395-405.
- Datsenko KA, Wanner BL (2000), Proc Natl Acad Sci U S A. 97: 6640-6645.
- Ehsani M, Fernández MR, Biosca JA, Julien A, Dequin S. (2009), Appl Environ Microbiol. 75(10):3196-205.
- Garg SK, Jain A. (1995), Bioresour Technol. 51:103-109.
- Gietz RD, Woods RA. (2002), Methods Enzymol. 350:87-96.
- Gietz RD, Schiestl RH. (2007), Nat. Protoc. 2:31-34.
- González E, Fernández MR, Marco D, Calam E, Sumoy L, Pares X, Dequin S, Biosca JA. (2010), Appl Environ Microbiol. 76(3):670-9.
- Guldener U, Heck S, Fielder T, Beinhauer J, Hegemann JH. (1996), Nucleic Acids Res. 24(13):2519-24.
- Gueldener U, Heinisch J, Koehler GJ, Voss D, Hegemann JH. (2002), Nucleic Acids Res. 30(6):e23.
- Guthrie & Fink, (2004) Methods in Enzymology, Volume 194, Guide to Yeast Genetics and Molecular and Cell Biology (Part A, 2004, Christine Guthrie and Gerald R. Fink (Eds), Elsevier Academic Press, San Diego, CA.
- Hahn-Hagerdal B, Karhumaa K, Jeppsson M, Gorwa-Grauslund MF. (2007), Adv Biochem Eng Biotechnol. 108:147-77.
- Hamacher T, Becker J, Gárdonyi M, Hahn-Hägerdal B, Boles E. (2002), Microbiology. 148(Pt 9):2783-8.
- Hasty et al. (1991), Mol Cell Biol 11:5586-91.
- Hatti-Kaul R, Törnvall U, Gustafsson L, Börjesson P. (2007), Trends Biotechnol. 25(3):119-24.
- Jin YS, Jeffries TW. (2004), Metab Eng. 6(3):229-38.
- Jin YS, Laplaza JM, Jeffries TW. (2004), Appl Environ Microbiol. 70(11):6816-25.
- Ji XJ, Huang H, Ouyang PK. (2011), Biotechnol Adv. 29(3):351-64.
- Larsson C, Nilsson A, Blomberg A, Gustafsson L. (1997), J Bacteriol. 179(23):7243-50.
- Larsson C, Påhlman IL, Gustafsson L. (2000), Yeast. 16(9):797-809.
- Liu M, Sergienko EA, GuoF, Wang J, Tittmann K, Hubner G, Furey W, Jordan F. (2011), Biochemistry. 40:7355-7368.
- Ma H, Kunes S, Schatz PJ, Botstein D. (1987), Gene. 58:201-2016.
- Magee RJ, Kosaric N. (1985), Adv Biochem Eng Biotechnol. 32:61-93.
- Miller (1992), A Short Course in Bacterial Genetics: A Laboratory Manual and Handbook for Escherichia coli and Related Bacteria, Cold Spring Harbor Laboratory Press, Cold Spring Harbor, New York.
- Nevoigt E, Fischer C, Mucha O, Matthäus F, Stahl U, Stephanopoulos G. (2007), Biotechnol Bioeng. 96(3):550-8.
- Ng CY, Jung M, Lee J, Oh M. (2012), Microbial Cell Factories. 11: 68-81.
- Nielsen DR, Yoon SH, Yuan CJ, Prather KL. (2010), Biotechnol J. 5(3):274-84.
- Portnoy VA, Scott DA, Lewis NE, Tarasova Y, Osterman AL, Palsson BØ. (2010), Appl Environ Microbiol. 76(19):6529-40.
- Raymond CK, Pownder TA, Sexson S. (1999), BioTechniques. 26:134-141.
- Romano P, Suzzi G. (1996), Appl Environ Microbiol. 62(2):309-15.
- Romano P, Suzzi G, Brandolini V, Menziani E, Domizio P. (1996), Lett Appl Microbiol. 22(4):299-302.
- Runquist D, Hahn-Hägerdal B, Rådström P. (2010), Biotechnol Biofuels. 3:5.
- Saloheimo A, Rauta J, Stasyk OV, Sibirny AA, Penttilä M, Ruohonen L. (2007), Appl Microbiol Biotechnol. 74(5):1041-52.
- Sambrook J et al. (1989) (2001), "Molecular Cloning: A Laboratory Manual" 2nd & 3rd Editions, Cold Spring Harbor Laboratory Press.
- Schaefer U, Boos W, Takors R, Weuster-Botz D., (1999), Anal. Biochem. 270: 88-96.
- Sedlak M, Ho NW. (2004), Yeast. 21(8):671-84.
- Smidt O, Preez JC, Albertyn J. (2012), FEMS Yeast Res. 12(1):33-47.
- Sunderland, MA., or Deshpande, Mukund V. (1992), Appl. Biochem. Biotechnol., 36:227.
- Syu MJ. (2001), Appl Microbiol Biotechnol. 55(1):10-8.
- Takasaki K, Shoun H, Yamaguchi M, Takeo K, Nakamura A, Hoshino T, Takaya N. (2004), J Biol Chem. 279(13):12414-20.
- Ui S, Takusagawa Y, Sato T, Ohtsuki T, Mimura A, Ohkuma M, Kudo T. (2004), Lett Appl Microobiol. 39(6):533-7.
- Wach A, Brachat A, Pohlmann R, Philippsen P. (1994), Yeast. 10:1793-1808.
- Wieczorke R, Krampe S, Weierstall T, Freidel K, Hollenberg CP, Boles E. (1999), FEBS Lett. 464(3): 123-8.
- Wu KJ, Saratale GD, Lo YC, Chen WM, Tseng ZJ, Chang MC, Tsai BC, Su A, Chang JS. (2008), Bioresour Technol. 99(17):7966-70.
- Zhou Z, Takaya N, Nakamura A, Yamaguchi M, Takeo K, Shoun H. (2002), J Biol Chem. 277(3):1892-6.

## Claims

1. A recombinant microorganism engineered for the production of 2,3-butanediol (BDO) wherein said microorganism overexpresses the following enzymes involved in the conversion of pyruvate into 2,3-butanediol:
a) a polypeptide catalysing the conversion of pyruvate into acetyl-CoA by overexpression of *pflA* and/or *pflB* genes encoding pyruvate formate lyase,
b) a polypeptide catalysing the conversion of said acetyl-CoA into acetaldehyde by overexpression of *mhpF* gene encoding acetaldehyde dehydrogenase,
c) a polypeptide catalysing the condensation of said acetaldehyde into acetoin by overexpression one gene chosen among *PDC1, PDC5* and *PDC6* encoding pyruvate decarboxylase, and
d) a butanediol dehydrogenase (BDH) catalysing the conversion of said acetoin into 2,3-butanediol.

2. The recombinant microorganism according to claim 1, wherein BDH is encoded by *budC* from *Klebsiella pneumoniae, BDH1* from *Saccharomyces cerevisiae,* or *BDH2* from *Saccharomyces cerevisiae.*

3. The recombinant microorganism of anyone of claims 1 to 2, wherein the cell is further modified to overexpress at least one gene encoding for: aldose reductase, xylitol dehydrogenase, xylulokinase, L-arabinol-4-dehydrogenase, L-xylulose reductase, hexose transporter and galactose transporter.

4. The recombinant microorganism of anyone of claims 1 to 3, wherein said microorganism is chosen among *Saccharomycetaceae* species or *Enterobacteriaceae* species.

5. A method of production of 2,3-butanediol (BDO) comprising the following steps:
- providing a recombinant microorganism according to anyone of the previous claims,
- cultivating the recombinant microorganism in a culture medium containing a source of carbon, and
- recovering the 2,3-butanediol.

## Patentansprüche

1. Rekombinanter Mikroorganismus, der zur Produktion von 2,3-Butandiol (BDO) konstruiert ist, wobei der besagte Mikroorganismus die folgenden Enzyme, die an der Umwandlung von Pyruvat in 2,3-Butandiol beteiligt sind, überexprimiert:
a) ein Polypeptid, das die Umwandlung von Pyruvat in Acetyl-CoA durch Überexpression der *pflA-* und/oder *pflB*-Gene, die Pyruvatformiatlyase kodieren, katalysiert,
b) ein Polypeptid, das die Umwandlung des besagten Acetyl-CoA in Acetaldehyd durch Überexpression des mhpF-Gens, das Acetaldehyddehydrogenase kodiert, katalysiert,
c) ein Polypeptid, das die Kondensation des besagten Acetaldehyds in Acetoin durch Überexpression eines Gens, das aus *PDC1, PDC5* und *PDC6* ausgewählt ist und das Pyruvatdecarboxylase kodiert, katalysiert, und
d) eine Butandioldehydrogenase (BDH), die die Umwandlung des besagten Acetoins in 2,3-Butandiol katalysiert.

2. Rekombinanter Mikroorganismus nach Anspruch 1, wobei die BDH durch *budC* von *Klebsiella pneumoniae, BDH1* von *Saccharomyces cerevisiae* oder *BDH2* von *Saccharomyces cerevisiae* kodiert wird.

3. Rekombinanter Mikroorganismus nach einem der Ansprüche 1 bis 2, wobei die Zelle weiter modifiziert wird, um mindestens ein Gen zu überexprimieren, das für folgende kodiert: Aldosereduktase, Xylitdehydrogenase, Xylulokinase, L-Arabinol-4-dehydrogenase, L-Xylulosereduktase, Hexosetransporter und Galaktosetransporter.

4. Rekombinanter Mikroorganismus nach einem der Ansprüche 1 bis 3, wobei der besagte Mikroorganismus aus der *Saccharomycetaceae*-Spezies oder der *Enterobacteriaceae*-Spezies ausgewählt ist.

5. Verfahren zur Produktion von 2,3-Butandiol (BDO), die folgenden Schritte umfassend:
- Bereitstellen eines rekombinanten Mikroorganismus nach einem der vorhergehenden Ansprüche,
- Kultivieren des rekombinanten Mikroorganismus in einem Kulturmedium, das eine Kohlenstoffquelle enthält, und
- Gewinnen des 2,3-Butandiols.

## Revendications

1. Micro-organisme recombinant conçu pour la production de 2,3-butanediol (BDO) dans lequel ledit micro-organisme surexprime les enzymes suivantes impliquées dans la conversion de pyruvate en 2,3-butanediol :
a) un polypeptide catalysant la conversion de pyruvate en acétyl-CoA par surexpression des gènes *pflA* et/ou *pflB* codant la pyruvate formate lyase,
b) un polypeptide catalysant la conversion dudit acétyl-CoA en acétaldéhyde par surexpression du gène *mhpF* codant l'acétaldéhyde déhydrogénase,
c) un polypeptide catalysant la condensation dudit acétaldéhyde en acétoïne par surexpression d'un gène choisi parmi *PDC1, PDC5* et *PDC6* codant la pyruvate décarboxylase, et
d) un butanediol déhydrogénase (BDH) catalysant la conversion dudit acétoïne en 2,3-butanediol.

2. Micro-organisme recombinant selon la revendication 1, dans lequel BDH est codé par *budC* de *Klebsiella pneumoniae, BDH1* de *Saccharomyces cerevisiae,* ou *BDH2* de *Saccharomyces cerevisiae.*

3. Micro-organisme recombinant selon l'une quelconque des revendications 1 à 2, dans lequel la cellule est en outre modifiée pour surexprimer au moins un gène codant pour : une réductase d'aldose, une xylitol déhydrogénase, une xylulokinase, une L-arabinol-4-déhydrogénase, une réductase de L-xylulose, un transporteur d'hexose et un transporteur de galactose.

4. Micro-organisme recombinant selon l'une quelconque des revendications 1 à 3, dans lequel ledit micro-organisme est choisi parmi des espèces *Saccharomycetaceae* ou des espèces *Enterobacteriaceae.*

5. Méthode de production de 2,3-butanediol (BDO) comprenant les étapes suivantes :
- la fourniture d'un micro-organisme recombinant selon l'une quelconque des revendications précédentes,
- la culture du micro-organisme recombinant dans un milieu de culture contenant une source de carbone, et
- la récupération de 2,3-butanediol.
